# EUROPEAN PATENT SPECIFICATION

(11) **EP 2 776 577 B1**
(45) Date of publication and mention of the grant of the patent: **11.01.2017**
(21) Application number: 12788123.3
(22) Date of filing: 07.11.2012
(51) Int. Cl.: C12N 15/10, C12N 1/06, C12Q 1/68

(54) **LYSIS METHOD AND LYSIS COMPOSITION**
LYSE-VERFAHREN UND LYSE-ZUSAMMENSETZUNG
PROCÉDÉ ET COMPOSITION DE LYSE

(30) Priority: 07.11.2011 EP 11188124
(43) Date of publication of application: 17.09.2014
(73) Proprietor: Qiagen GmbH, 40724 Hilden (DE)
(72) Inventor: HIMMELREICH, Ralf, 55131 Mainz (DE); HOLZER, Eva, 40724 Hilden (DE); GROSSHAUSER, Gerd, 40724 Hilden (DE); WENDE, Andy, 40724 Hilden (DE)
(74) Representative: Roth, Carla
(86) International application number: PCT/EP2012/004632
(87) International publication number: WO 2013/068107

(56) References cited:
- WO-A1-2008/000344
- US-A1- 2009 018 323
- US-A1- 2011 177 516
- KUSONWIRIYAWONG C ET AL: "Evaluation of pH-dependent membrane-disruptive properties of poly(acrylic acid) derived polymers", EUROPEAN JOURNAL OF PHARMACEUTICS AND BIOPHARMACEUTICS, ELSEVIER SCIENCE PUBLISHERS B.V., AMSTERDAM, NL, vol. 56, no. 2, 1 September 2003 (2003-09-01), pages 237-246, XP004453358, ISSN: 0939-6411, DOI: 10.1016/S0939-6411(03)00093-6
- MURTHY N ET AL: "The design and synthesis of polymers for eukaryotic membrane disruption", JOURNAL OF CONTROLLED RELEASE, ELSEVIER, AMSTERDAM, NL, vol. 61, no. 1-2, 27 August 1999 (1999-08-27), pages 137-143, XP004362971, ISSN: 0168-3659, DOI: 10.1016/S0168-3659(99)00114-5
- YESSINE M-A ET AL: "Characterization of the membrane-destabilizing properties of different pH-sensitive methacrylic acid copolymers", BIOCHIMICA ET BIOPHYSICA ACTA. BIOMEMBRANES, AMSTERDAM, NL, vol. 1613, no. 1-2, 27 June 2003 (2003-06-27), pages 28-38, XP004433589, ISSN: 0005-2736, DOI: 10.1016/S0005-2736(03)00137-8

## Description

### FIELD OF THE INVENTION

The present invention inter alia relates to a method for lysing a sample wherein the lysed sample can be used directly for amplifying nucleic acids comprised in the lysed sample. Furthermore, the present invention provides a lysis composition which eliminates inhibitors of amplification reactions, in particular PCR inhibitors. Thus, samples that are lysed with a respective lysis composition can be used directly in an amplification reaction. There is no need to purify the nucleic acids in advance.

### BACKGROUND OF THE INVENTION

There is currently a great demand for rapid analytical methods processing a biological sample and detecting and analyzing biomolecules comprised therein. The prerequisite for such an analytical method is that the biomolecules to be analysed, e.g. nucleic acids, are made available for the analytical method in such a way that the biomolecules of interest are provided in an amount capable of being analyzed and in a sufficient purity for the intended analytical method. For analysing, e.g. detecting nucleic acids, e.g. a widely used method is the polymerase chain reaction (PCR), by which the nucleic acid of interest is amplified. PCR is an analytical method, since it is possible, by selecting specific primers, to identify the presence of corresponding sequences in the population of nucleic acids comprised in the sample. Furthermore, the PCR amplificates themselves may be the subject of further analyses e.g. they can be detected, sequenced, identified or analysed otherwise. Isothermal nucleic acids amplification and detection reactions are well-known alternatives to PCR. Examples for this category of analytical methods are LAMP (loop mediated isothermal amplification), RPA (recombinase polymerase amplification), tHDA (helix dependent amplification), NEAR (nicking enzyme amplification reaction), TMA (transcription mediated amplification) and NASBA (nucleic acid sequence based amplification).

It is desirable to employ inexpensive, time-saving methods for such an analysis since it is frequently the case that larger amounts of different samples have to be processed simultaneously. Therefore, it is desireous to be able to analyse the target biomolecules without purifying them first and hence to be able to perform the analytical method with the crude lysate (which optionally may be cleared) which comprises the biomolecules of interest. By overcoming the need to isolate the biomolecules first, time and costs can be saved. To be able to use the crude lysate in an analytical method such as an amplification reaction, the lysis conditions must be carefully chosen because if unsuitable conditions or buffer systems are chosen, the analytical method can not be carried out with sufficient performance. This particularly, if increased amounts of the lysed sample are supposed to be transferred into the analytical method. The higher the amount of lysed sample, the higher the amount of potential inhibitors comprised therein, that could disturb the performance of the analysis method such as e.g. an amplification reaction. Therefore, it is important to reduce or neutralize contaminants that could inhibit the intended analytical method.

A suitable lysis buffer which enables to use the crude lysate directly in an amplification reaction is e.g. described in US 2011/0177516. The lysis buffer disclosed therein comprises binders or thickeners such as PVP which bind to or neutralize PCR inhibitors comprised in the lysate. The polymer is employed to prevent an inhibition of the subsequent analytical methods by the unspecific complexing of potential inhibitors, so that the lysate may be used immediately subsequently for the analysis.

However, there is still a need to provide a method and means that are suitable for providing a lysate that can be directly used in an analytical method such as an amplification method. In particular, there is a need to provide a lysis method which allows to remove contaminants that may potentially interfere with the intended analytical method, preferably more efficiently.

Therefore, it is the object of the present invention to provide a further method for providing a lysate that can be used directly in an analytical method such as an amplification method.

### SUMMARY OF THE INVENTION

The present invention is based on the finding that a sample can be lysed efficiently and that the provided lysate is suitable for being used directly in an analytical method such as an amplification method, if the sample is contacted for lysis with at least one non-ionic surfactant and at least one water-soluble polyanionic polymer, wherein said polymer is (i) a polymer consisting of carboxylate-containing monomers that are selected from acrylic acid, methacrylic acid and/or maleic acid, or (ii) a polymer consisting of carboxylate-containing monomers that are selected from acrylic acid, methacrylic acid and/or maleic acid and additional polar monomers without a carboxylate function that are selected from lactide and/or vinylpyrrolidone. As shown in the examples, using a respective lysing procedure renders a lysate that can be used directly in an amplification reaction. Inhibitors of the amplification reaction are advantageously neutralised, e.g. complexed, due to the used lysis method. Here, in particular said water-soluble polyanionic polymer is decisive for reducing the inhibiting effect of amplification inhibitors that are comprised in the lysed sample. Respective inhibitors are e.g. released from the biological sample during lysis. Thus, the incorporation of said water-soluble polyanionic polymer during lysis has the effect that the subsequent analysis method such as e.g. an amplification reaction shows an improved performance if said water-soluble polyanionic polymer is incorporated into the lysis solution compared to if said polymer is not being incorporated into the lysis solution. That the amplification performance can be improved was particularly surprising because water-soluble polyanionic polymers such as e.g. polyacrylic acid are in fact known to act as PCR inhibitors and thus were known to have the opposite effect.

Hence, that respective water-soluble polyanionic polymers as defined above, such as polyacrylic acid, can be used to reduce the inhibitory effect of PCR inhibitors thereby improving the performance of the amplification reaction was highly unexpected. Furthermore, as shown by the examples, using said water-soluble polyanionic polymer for neutralizing amplification inhibitors provides results that are improved compared to prior art methods. In particular compared to binders/thickeners such as PVP that were used in the prior art to deplete inhibitors, the water-soluble polyanionic polymers that are used according to the present invention show a significantly better depletion performance with numerous different sample types. The term "depletion" of inhibitors and similar terms used herein are used in a broad sense and do not include a limitation regarding the mode of action. In particular, said terms include e.g. unspecific binding, e.g. complexing, of inhibitors by the water-soluble polyanionic polymers and the like. An inhibitor released from a biological sample during lysis is depleted in this sense, if the subsequent analysis method such as an amplification reaction shows an improved performance due to the addition of said water-soluble polyanionic polymer during lysis compared to when said polymer is not used during lysis.

According to a first aspect, the present invention pertains to a method for lysing a sample to provide a lysis mixture that is suitable for being directly used in a method for analysing nucleic acids. In said lysis method, the sample is contacted with
(a) at least one non-ionic surfactant,
(b) at least one water-soluble polyanionic **polymer,** wherein said polymer is (i) a polymer consisting of carboxylate-containing monomers that are selected from acrylic acid, methacrylic acid and/or maleic acid, or (ii) a polymer consisting of carboxylate-containing monomers that are selected from acrylic acid, methacrylic acid and/or maleic acid and additional polar monomers without a carboxylate function that are selected from lactide and/or vinylpyrrolidone,
to provide a lysis mixture,
wherein the sample is contacted prior to, during or after lysis with at least one magnetic solid support which binds precipitates, thereby forming a complex with the precipitates.

As described above, it was surprisingly found that the addition of a water-soluble polyanionic polymer as defined above provides a lysis mixture that can be directly used in an analytical method such as e.g. a nucleic acid amplification reaction. In fact, the lysis mixture can even be added in rather large amounts into the amplification reaction without significant inhibition of the subsequent analysis method. It is a particular advantage that rather large amounts of lysed sample can be transferred into or used in the analysis method, as thereby the sensitivity of the subsequent analytical method can be improved because e.g. more nucleic acids are transferred with the lysis mixture into the analytical method.

According to a second aspect, the present invention pertains to a nucleic acid amplification method, comprising
a) lysing a sample comprising nucleic acids according to the method of the first aspect of the present invention and
b) using at least a portion of the lysis mixture in an amplification reaction.

As described above, the used lysis conditions provide a lysate that can be used directly in a nucleic acid amplification reaction. There is no need to purify the nucleic acids first. The lysate may be optionally cleared according to the teachings of the present invention.

According to a third aspect, a lysis solution is provided, which lysis solution is suitable to provide a lysis mixture that can be directly used in a nucleic acid amplification, and comprises
(a) at least one non-ionic surfactant,
(b) at least one water-soluble polyanionic polymer comprising carboxylate-containing monomers, wherein the at least one water-soluble polyanionic polymer or mixture of water-soluble polyanionic polymers is comprised in the lysis solution in a concentration of 0.01% (w/v) to 1% (w/v), and wherein said polymer is
   (i) a polymer consisting of carboxylate-containing monomers that are selected from acrylic acid, methacrylic acid and/or maleic acid, or
   (ii) a polymer consisting of carboxylate-containing monomers that are selected from acrylic acid, methacrylic acid and/or maleic acid and additional polar monomers without a carboxylate function that are selected from lactide and/or vinylpyrrolidone,
   optionally wherein said polymer is a polymer selected from polyacrylic acid, polymaleic acid, polymethacrylic acid, poly(acrylic acid-co-maleic acid), poly(acrylic acid-co-lactide), poly(acrylic acid-co-vinylpyrrolidone), poly(maleic acid-co-lactide), poly(maleic acid-co-vinylpyrrolidone), poly(acrylic acid-co-maleic acid-co-lactide) and poly(acrylic acid-co-maleic acid-co-vinylpyrrolidone).

This lysis solution can be advantageously used in the methods according to the first and second aspect of the present invention to provide a lysed sample.

Further disclosed is a kit which comprises a lysis composition according to the third aspect of the present invention, and optionally one or more reagents for performing an amplification reaction.

Other objects, features, advantages and aspects of the present application will become apparent to those skilled in the art from the following description and appended claims 1 to 15, which define the scope of the present invention.

### DETAILED DESCRIPTION OF THIS INVENTION

According to a first aspect, the present invention pertains to a method for lysing a sample to provide a lysis mixture that is suitable for being directly used in a method for analysing nucleic acids, in particular in a nucleic acid amplification method. In said lysis method, the sample is contacted with
(a) at least one non-ionic surfactant,
(b) at least one water-soluble polyanionic polymer, wherein said polymer is
   (i) a polymer consisting of carboxylate-containing monomers that are selected from acrylic acid, methacrylic acid and/or maleic acid, or
   (ii) a polymer consisting of carboxylate-containing monomers that are selected from acrylic acid, methacrylic acid and/or maleic acid and additional polar monomers without a carboxylate function that are selected from lactide and/or vinylpyrrolidone,
   optionally wherein said polymer is a polymer selected from polyacrylic acid, polymaleic acid, polymethacrylic acid, poly(acrylic acid-co-maleic acid), poly(acrylic acid-co-lactide), poly(acrylic acid-co-vinylpyrrolidone), poly(maleic acid-co-lactide), poly(maleic acid-co-vinylpyrrolidone), poly(acrylic acid-co-maleic acid-co-lactide) and poly(acrylic acid-co-maleic acid-co-vinylpyrrolidone),
to provide a lysis mixture,
wherein the sample is contacted prior to, during or after lysis with at least one magnetic solid support which binds precipitates, thereby forming a complex with the precipitates.

As described above, the resulting lysis mixture which comprises the lysed sample can be used directly in a method for analysing nucleic acids, such as in particular an amplification reaction. Contaminants that are comprised in the lysis mixture due to the lysis of the sample such as in particular proteoglycans, proteins, metabolites and sugars are advantageously inactivated by the water-soluble polyanionic polymer. Thereby, a lysis mixture is provided that can be used directly in a nucleic acid analysis method thereby overcoming the need to isolate and purify the nucleic acids first. Hence, "directly" in this respect means that it is not necessary to isolate and in particular to purify the nucleic acids comprised in the lysis mixture prior to performing the subsequent nucleic acid analysis method which preferably is an amplification method. Therefore, no nucleic acid isolation or purification is performed prior to performing the nucleic acid analysis method. However, as described below it is within the scope of the present invention to further process the lysis mixture prior to subjecting an aliquot thereof to the nucleic acid analysis method. E.g. the lysis mixture may be cleared and an aliquot of the cleared lysate is then used in the nucleic acid analysis method which preferably is an amplification reaction. Furthermore, the obtained lysis mixture may also be treated with enzymes such as RNases, DNases and/or a reverse transcriptase prior to performing the nucleic acid analysis method. Due to the efficient inactivation of inhibitors comprised in the lysis mixture, increased amounts of lysis mixture can be used in the subsequent nucleic acid analysis method. This is an important advantage as the sensitivity is increased because larger amounts of nucleic acids are subjected to the analysis method. This higher amount of nucleic acids may also compensate for a potential inhibition of the analytical reaction such as an amplification reaction by contaminants comprised in the lysed sample.

The term "lysis" as used herein refers to the disruption, degradation and/or digestion of a sample. In a respective lysis step, biomolecules such as in particular nucleic acids can be released from cells or can be freed from other sample additives such as e.g. proteins. Herein, we refer to a respective step to disrupt, degrade and/or digest a sample generally as lysis step, irrespective of whether biomolecules such as in particular nucleic acids are released from cells or whether the lysis is performed in order to release biomolecules such as nucleic acids e.g. from proteins or other substances comprised in the sample. Hence, the sample may comprise cells or may comprise no or only minor amounts of cells as is e.g. the case with blood plasma.

Subsequently, we will explain the individual additives that are used for lysis and preferred embodiments thereof in detail.

As additive (a), at least one non-ionic surfactant is used in the lysis method according to the present invention which aids the lysis of the sample and which dissolves protein aggregates. Also a mixture of non-ionic surfactants can be used. Substances which are suitable for this purpose are, in principle, any non-ionic surfactant which has no adverse effect on the nucleic acids to be examined. The non-ionic surfactant preferably is selected from the group consisting of polyoxyethylene fatty alcohol ethers, polyoxyethylene alkylphenyl ethers, polyoxyethylene-polyoxypropylene block copolymers, polysorbates and alkylphenol ethoxylates, preferably polyoxyethylene fatty alcohol ethers, polysorbates and/or polyoxyethylene alkyl-phenyl ethers.

The term "fatty alcohol" in particular means for the purposes of the present invention alcohols having a chain length of from 6 to 22 carbon atoms, preferably 8 to 20 carbon atoms, preferentially 10 to 18 carbon atoms, particularly preferably 12 to 18 carbon atoms. Preference is in particular given to alcohols having 12, 14, 16 or 18 carbon atoms. Although the fatty alcohols may be mono- or polyunsaturated, they are preferably saturated fatty alcohols. The term "polyoxyethylene" in particular means for the purposes of the present invention an HO-(CH₂CH₂O)ₙ unit, with n being preferably an integer from 2 to 150, further preferably from 4 to 120, still further preferably from 8 to 80, and most preferably an integer selected from 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 and 50.

Preferred examples of suitable polyoxyethylene fatty alcohol ethers are polyethoxylated lauryl, cetyl, oleyl, or stearyl alcohols which may be used alone or as mixture. According to a preferred embodiment of the invention, the at least one polyoxyethylene fatty alcohol ether comprises a fatty alcohol moiety having from 6 to 22 carbon atoms and a polyoxyethylene moiety having from 2 to 150 (CH₂CH₂O units. Preferably, the polyoxyethylene fatty alcohol ether is selected from the group consisting of polyoxyethylene lauryl ether, polyoxyethylene cetyl ether (Brij-58), polyoxyethylene stearyl ether and/or polyoxyethylene oleyl ether. As alkylglucoside, preferably a non-ionic surfactant from the group of the polysorbates, preferably polysorbate 20 (Tween 20), polysorbate 40 or polysorbate 80, more preferred polysorbate 20 is used. Preferred examples of polyoxyethylene alkyl phenyl ethers include Tergitol, Triton X-100 und Nonidet P-40 (nonylphenylpolyethylenglycol).

Preferably, at least two respective non-ionic surfactants are used as additive (a). According to one embodiment, additive (a) is selected from the group consisting of Tween 20, Tergitol, Triton X-100, Brij-58 and Nonidet P-40. Preferably, at least two respective non-ionic surfactants are added as additive (a). As described subsequently, preferably additive (a) is added in form of a lysis composition such as a lysis solution. The concentration of the non-ionic surfactant or the mixture of non-ionic surfactants in a respective lysis composition is preferably selected from 0.05% (v/v) to 5% (v/v), 0.1% (v/v) to 2% (v/v), 0.2% (v/v) to 1.5% (v/v) and 0.4% (v/v) to 1% (v/v). In the lysis mixture the concentration may be e.g. 0.05% to 1.2%, preferably 0.2 to 0.9% or 0.45%. Preferably, the overall concentration of non-ionic detergent(s) in the lysis mixture is ≤ 5%, preferably ≤ 3% (v/v), more preferably ≤ 2% (v/v). Preferably, Tween 20 and/or Nonidet P-40, preferably a mixture of both, are used as additive (a).

Furthermore, in the lysis method according to the present invention the sample is contacted with at least one water-soluble polyanionic polymer as defined above as additive (b). Thus, the incorporation of the water-soluble polyanionic polymer during lysis has the effect that the subsequent analysis method such as e.g. an amplification reaction shows an improved performance when said polymer is incorporated into the lysis solution compared to when said polymer is not incorporated into the lysis solution. It is assumed that the water-soluble polyanionic polymer prevents or reduces the inhibition by unspecifically complexing potential inhibitors. Which compounds act as inhibitors also depends on the analytical method that is subsequently performed. E.g. typical inhibitors of a nucleic acid amplification reaction which usually originate from the sample comprising the nucleic acids include but are not limited to proteoglycans, proteins, metabolites and sugars. As shown in the examples, the addition of the water-soluble polyanionic polymer during lysis significantly improves the performance of the amplification reaction.

Preferred water-soluble polyanionic polymers, including copolymers, comprising carboxylate-containing monomers, are acrylic acid, methacrylic acid and/or maleic acid. According to one embodiment said polymer comprises only two types of monomers. Preferably, only one type of monomer is comprised in the polymer.

In a preferred embodiment the polymer is polyacrylic acid, polymaleic acid or poly(acrylic acid-co-maleic acid). Most preferably polyacrylic acid is used. As shown in the examples, polyacrylic acid is very efficient in preventing the inhibition of amplification reactions. If a copolymer is used, it may additionally comprise polar monomers which do not contain carboxylate. Examples of respective polar monomers encompass lactide and/or vinylpyrrolidone. According to one embodiment, said polymer comprises only type of the respective polar monomers. According to one embodiment the copolymer is poly(acrylic acid-co-lactide), poly(acrylic acid-co-vinylpyrrolidone), poly(maleic acid-co-lactide), poly(maleic acid-co-vinylpyrrolidone), poly(acrylic acid-co-maleic acid-co-lactide) or poly(acrylic acid-co-maleic acid-co-vinylpyrrolidone). Preferably, the copolymer is a block copolymer.

Also mixtures of water-soluble polyanionic polymers can be used as additive (b).

The molecular weight of said water-soluble polyanionic polymer is preferably chosen such that it is present in a solved form in the lysis mixture. The average molecular weight of said at least one water-soluble polyanionic polymer is preferably selected from 2,000 Da to 300,000 Da, 50,000 Da to 300,000 Da, 100,000 Da to 300.000 Da and 200,000 Da to 300,000 Da. Further suitable ranges include 2,000 Da to 500,000 Da, 10,000 to 450,000 Da, 25,000 to 400,000 Da, 35,000 to 350,000 Da, 50,000 Da to 300,000 Da, 75,000 to 300,000 Da, 100,000 Da to 300.000 Da, 150,000 to 300,000 Da and 200,000 Da to 300,000 Da. In a preferred embodiment the average molecular weight is approx. 250,000 Da. Preferably, said at least one water-soluble polyanionic polymer or mixture of water-soluble polyanionic polymers is provided in form of a lysis composition, preferably a lysis solution in a concentration that is selected from 0.01% (w/v) to 1% (w/v), 0.02% (w/v) to 0.5% (w/v), 0.025% (w/v) to 0.3% (w/v), 0.03% (w/v) to 0.3% (w/v), 0.035% (w/v) to 0.2% (w/v) 0.04% (w/v) to 0.2% (w/v), 0.05% (w/v) to 0.15% (w/v) and 0.05% (w/v) to 0.1% (w/v). Preferably, it is comprised in the lysis composition in a concentration selected from 0.025% (w/v) to 0.15% (w/v), 0.03% (w/v) to 0.1% (w/v), 0.03% (w/v) to 0.075% (w/v) and 0.035% (w/v) to 0.06% (w/v). Most preferred, it is comprised in the lysis composition that is added to the sample in a concentration of approx. 0.05% (w/v).

Furthermore, according to one embodiment, the sample is additionally contacted with at least one proteolytic enzyme and/or a cell wall digesting enzyme as additive (c). Also mixtures of respective enzymes can be used. The cell wall digesting enzyme is preferably selected from the group consisting of lysozyme, lyticase, zymolyase and pektinases. Exemplary proteolytic enzymes in particular include but are not limited to subtilisins, subtilases, alkaline serine proteases, proteinases and the like. Subtilases are a family of serine proteases, i.e. enzymes with a serine residue in the active side. Subtilisins are bacterial serine proteases that have broad substrate specificities. Exemplary subtilisins include but are not limited to proteinase K, proteinase R, proteinase T, subtilisin, subtilisin A, QIAGEN Protease and the like. Discussions of subtilases, subtilisins, proteinase K and other proteases may be found, among other places in Genov et al., Int. J. Peptide Protein Res. 45: 391-400, 1995. Preferably, the proteolytic enzyme is used under heating and/or agitation. It is preferred to employ a thermophilic proteinase, especially preferably a thermophilic proteinase selected from among proteinase K or subtilisin. It is very especially preferred to use a thermophilic proteinase K. The one or more enzymes that are used as additive (c) can be added directly to the sample or may also be provided together with additives (a) and (b) in a lysis composition, which preferably is a lysis solution. The proteinase is preferably employed in such an amount that it provides from 0.5 to 10 µunits, preferably from 1.5 to 4 µunits (international units) per milliliter of lysis composition.

According to one embodiment, the sample is contacted for lysis additionally with an additive (d), which is at least one chelating agent for divalent cations. Preferably, additive (d) is also incorporated into a lysis composition together with additives (a), (b) and optionally (c). Suitable chelating agents include but are not limited to diethylenetriaminepentaacetic acid (DTPA), ethylenedinitrilotetraacetic acid (EDTA), ethylene glycol tetraacetic acid (EGTA) and N,N-bis(carboxymethyl)glycine (NTA). According to a preferred embodiment, EDTA is used. As used herein, the term "EDTA" indicates inter alia the EDTA portion of an EDTA compound such as, for example, K₂EDTA, K₃EDTA or Na₂EDTA. Using a chelating agent such as EDTA has the advantageous effect that nucleases such as DNases and RNases are inhibited. The chelating agent is comprised in the resulting lysate in a concentration, wherein it does not inhibit the subsequent downstream reaction, e.g. an amplification reaction. The chelating agent preferably is employed in a concentration of 0.5mM to 5mM, preferably 0.75mM to 2mM, most preferred 1 mM to 1.5mM in the lysis composition. Preferably, EDTA is used.

According to one embodiment, the sample is contacted for lysis additionally with an additive (e), which is at least one buffer substance. Preferably, additive (e) is also incorporated into a lysis composition together with additives (a), (b) and optionally (c) and (d). The buffer substance should be capable of buffering the lysis composition which preferably is a lysis solution at a pH range that is compatible with the pH range that is required for performing the subsequent analytical method which preferably is an amplification method. This, as the pH of the lysis mixture is strongly effected by the composition that is used for lysis. Preferably, a buffer is used in the lysis composition so that the pH of the composition is between 7.5 and 11, more preferred 7.5 to 9. Respectively buffered lysis compositions, preferably lysis solutions, are particularly suitable for providing a lysis mixture that is suitable for being directly used in a nucleic acid amplification reaction. The pH buffer substance can be comprised in the lysis composition in a concentration of 5mM to 100mM, preferably 10mM to 80mM, more preferably 10-50 mM. Preferably, the buffer substance is selected from the group consisting of Tris, MOPS, HEPES, borate or phosphate. Preferably, the buffer substance is selected from Tris or borate.

The additives (a), (b) as well as (d) and (e) (if used) are preferably provided in form of a lysis composition. The sample can then be conveniently contacted with said lysis composition. Furthermore, also additive (c) can be comprised in the lysis composition. However, it is also within the scope of the present invention to contact the sample separately with additive (c). Preferably, a lysis solution is used as lysis composition. The term "solution" as used herein in particular refers to a liquid composition, preferably an aqueous composition. It may be a homogenous mixture of only one phase but it is also within the scope of the present invention that a solution comprises solid additives such as e.g. precipitates.

A particularly preferred lysis solution comprises
- as additive (a) a mixture of two non-ionic detergents, preferably selected from polyoxyethylene fatty alcohol ethers, polysorbates and/or polyoxyethylene alkyl phenyl ethers, more preferred from polysorbate 20 (Tween 20) and nonylphenylpolyethylenglycol (Nonidet P-40), wherein preferably, each non-ionic detergent is comprised in a concentration of 0.2% (v/v) to 0.6% (v/v), preferably 0.4% (v/v) to 0.5% (v/v);
- as additive (b), the water-soluble polyanionic polymer, as defined above and in claim 1, which preferably is or comprises polyacrylic acid, in a concentration of 0.01% (w/v) to 0.5% (w/v), preferably 0.03% (w/v) to 0,2% (w/v);
- optionally a proteolytic enzyme such as proteinase K as additive (c);
- as additive (d) EDTA in a concentration below 1.5mM; and
- as additive (e) a buffer substance, preferably TRIS, in a concentration of 7.5mM to 50mM, preferably 10mM.

The pH value of the respective lysis solution preferably is approx. between 7.5 and 10, preferably 7.5 and 9.0, preferentially at pH 8.0 to 8,5. The described lysis solution is in particular compatible for performing an amplification reaction such as a PCR as it does not comprise additives in a concentration that would substantially inhibit a respective amplification reaction.

A lysis of the sample is particularly effective when the lysis mixture is incubated for a time sufficient to achieve lysis. Preferably, lysis is assisted by heating and/or agitation. Preferably, the lysis mixture is incubated at elevated temperatures to ensure an efficient lysis. Preferably, lysis is assisted by a heating step. For this purpose, the lysis mixture comprising the sample and the lysis solution is heated preferably for at least 3min, preferably at least 5min to a temperature of at least 90°C, preferably at least 95°C. Such a heating step is also referred to as boiling lysis. Preferably, the heated sample is then cooled down to a temperature below 50°C, more preferably it is cooled down at least to room temperature. If the lysis solution comprises and/or is used in combination with a proteolytic enzyme, it is preferred to incubate the lysis mixture first at temperatures suitable for the proteolytic enzyme to work, e.g. at a temperature below 70°C, preferably below 65°C, more preferably below 60°C, preferably at a temperature selected from the ranges 40°C to 60°C, more preferred 50°C to 60°C, preferably combined with agitation. After said incubation, the heating step at higher temperatures (e.g. at least 90°C) is performed. This sequence of process steps for achieving an efficient lysis of the sample has the advantage that the sample is efficiently lysed and that the proteolytic enzyme is denatured after it has been added to the digestion of the sample.

The lysis can be assisted by further measures such as e.g. further mechanical, chemical, physical or enzymatic actions on the sample. Examples of respective measures that can be performed to assist the lysis process include but are not limited to grinding the sample in a bead mill, sonication, surface acoustic waves (SAW), repeated cycles of freezing and thawing, the addition of further detergents and/or the addition of further protein degrading compounds. Furthermore, further additives can be incorporated into the lysis mixture that protect the encompassed nucleic acids from degradation. E.g. suitable DNase and/or RNase inhibitors can be added. The incorporation of RNase inhibitors is particularly recommended if RNA is the nucleic acid of interest as RNA is very susceptible to degradation. Suitable protective agents are known in the prior art and, thus, do not need any detailed description here. As the lysed sample is directly subjected to the analytical method such as e.g. an amplification reaction, it is important to ensure that the performed lysis does not introduce one or more inhibitors of the subsequent analysis method in a concentration that would inhibit the performance of the subsequent analytical method to an extent that the analytical method can not be adequately performed. The tolerable concentration depends on the analytical method to be performed, e.g. the used enzymes and their susceptibility to inhibitors and the sensitivity that is required for the adequate performance of the analytical methods. These parameters can also vary from sample to sample.

Furthermore, the sample may be pretreated prior to contacting the sample with additives (a) and/or (b). The type of pretreatment also depends on the type of sample that is processed. According to one embodiment, prior to lysis, nucleic-acid-containing cells are enriched from a biological sample, such as for example blood. A variety of methods is available to the skilled person for this purpose, and these methods are in principle known per se in expert circles. Such suitable methods include for example the lysing or destroying of undesired cells in the mixture, the addition of surfaces onto which the cells of interest to be lysed adsorb or bind, filtration, sedimentation, or centrifugation steps or a combination of a plurality of these methods, without being limited thereto. The preferred object is to separate the cells which contain the genetic material to be analysed from other cells or contaminating additives, or to enrich them with respect to the other additives.

According to one embodiment, blood samples are first treated with an erythrocyte lysis buffer so that the erythrocytes in the sample are lysed. Then, the remaining white blood cells are separated from the lysis mixture by centrifugation. The supernatant is discarded and the white blood cells are immediately available for further processing using the lysis method according to the present invention. Said lysis buffer serves exclusively for lysing erythrocytes which are present in blood. Any erythrocyte lysis buffer can be used for this purpose, without limiting the invention thereby. One suitable example is the lysis buffer ELB1 (320 mM sucrose, 50 mM Tris/Cl pH 7.5, 5 mM MgCl₂, 1% Triton X-100) or ELB2 (155 mM NH₄Cl, 10 mM KHCO₃).

According to one embodiment, the sample is brought into contact with a surface onto which the cells to be lysed adsorb or bind. Thereafter, the remaining constituents of the sample are largely removed by a separation method. To this end, the sample is brought into contact with a suitable surface onto which the cells adsorb or bind. Examples of such surfaces which are suitable for this purpose are small spheres, also known as beads, for example made of glass, silica, polymers or coated beads, preferably magnetic beads. However, it is also possible to modify the surfaces of the consumables such as, for example, reaction vessels, reaction filters, filter columns, spin filter minicolumns, membranes, frits, glass fibre fabric, dishes, tubes, (pipette) tips or wells of multiwell plates for the binding. In an especially preferred embodiment, magnetic beads are used. For the lysis of erythrocytes, a surface can be introduced in the form of modified magnetic particles. The white blood cells adsorb onto the surfaces and can be enriched by magnetic separation. Beads which are suitable for said purposes may comprise any type of magnetic beads known to date in which a magnetic core is coated with a glass or polymer coating and which on their surface bear groups which make possible an unspecific attachment or binding of nucleic-acid-containing cells onto the beads. It is preferred to employ those beads which are hydrophilic on their surface, for example which bear acid groups, preferably carboxylic acid groups, phosphoric acid groups or sulfuric acid groups, or their salts, more preferably carboxylic acid groups or their salts. For the abovementioned groups it is possible to be bound directly to the surface or to be part of the polymer which forms the surface coating, to be bound to the surface via spacer molecules or to be parts of a compound which is bound to the surface of the beads. In one embodiment, the beads bear on their surface a total charge which is weakly negative in overall terms, since the cells are bound particularly effectively when such conditions prevail. A neutral to weakly positive charge of the beads is also possible. Examples of suitable carboxylated polymers which are suitable as coating material for the beads and which provide a surface which is suitable for the invention are described in detail in the German patent application DE 10 2005 040 259.3. Examples of compounds which may be bound to the surface are glycine, hydrazine, aspartic acid, 6-aminocaproic acid, NTA (nitrilotriacetic acid), polyacrylic acid (PAA), glycerin, diglyme (diethylene glycol dimethyl ether), glyme (dimethoxyethane), pentaerythritol, toluene or combinations of these, without being limited thereto. Likewise suitable magnetic beads are those which are described in the German patent application DE 10 2005 058 979.9. Such suitable magnetic beads are commercially available. Further examples of suitable beads are silica beads, such as, for example, MagAttract Suspension-B, MagAttract Suspension-G (all by Qiagen). The cells are brought into contact with the magnetic beads over a sufficiently long period of time, i.e. a period of time which suffices to allow the cells to bind/attach themselves to the beads. Such a period of time should be at least 30 s, preferably at least 1 min, further preferably at least 3 min. If magnetic particles are employed in the lysis of the erythrocytes, a magnetic separation may be performed prior to removing the lysate for the subsequent detection reaction, in order to avoid carry-over of magnetic particles. However, beads may remain in the mixture, but should preferably not be carried over into the analysis method. DNA and RNA are present in the lysate in free form because the lysate does not have any properties which support the binding of nucleic acid to the beads.

After the attachment/binding of the cells to the magnetic particles, the cells can be collected or separated from the medium which surrounds the cells by applying a magnetic field to the vessel in which the cells together with the beads are located. In a preferred embodiment, a magnet is applied externally to the vessel in which the cells and the magnetic beads are located, and the remainder of the sample is removed from the vessel, for example decanted off, or removed with the aid of a suitable device, for example drawn off with the aid of a pipette. The magnetic particles together with the cells can optionally be resuspended in a suitable wash medium and thereby washed, where after a magnetic field is again applied to the vessel and the wash medium is again removed from the vessel. The present method therefore provides a particularly gentle handling of the cells.

After the enrichment of the nucleic-acid-containing cells to be lysed, the collected cells are lysed using the method according to the present invention as described in detail hereinabove. When the cells are contacted with a lysis solution as described above, wherein said lysis solution comprises at least additives (a) and (b) and optionally one or more of additives (c), (d) and (e), the resulting lysis mixture is incubated for a period of from 1 min to 3 h, preferably from 2 min to 1 h, more preferably from 3 to 20 min and most preferably 5 min at a temperature of from room temperature to below 100 °C, preferably at least 60 °C, more preferably at least 70 °C, further preferably at least 80 °C and most preferably 95 °C. As described above, also a two-step incubation may be performed. Thus, for example, it might be possible to incubate the lysis mixture first for at least 5min, preferably at least 10 min at 50 °C to 60°C and thereafter for at least 3min, preferably at least 5 min at 95 °C. The latter embodiment is particularly suitable if a proteolytic enzyme such as a proteinase, preferably proteinase K is used as additive (c).

In the present invention, the sample is contacted prior to, during or after lysis with at least one magnetic solid support which binds precipitates, thereby forming a complex with the precipitates. Further disclosed herein is that after the incubation step, the lysis mixture may, in an optional step, be further processed, e.g. in order to clear the lysis mixture. E.g. the lysis mixture can be centrifuged in order to separate insoluble cell additives from the lysis mixture. Respective insoluble additives such as in particular precipitates may also be removed using other means e.g. by using a filter or by adding a solid support which binds precipitates. Suitable embodiments are described below.

Precipitates that form during lysis are a major source of contaminations, in particular when processing a cell containing sample. Respective precipitates are in particular formed when the sample is heated during lysis. The carry-over of precipitates from the lysed sample into the analytical method such as e.g. an amplification reaction or a detection reaction can disturb said analysis method and/or the interpretation of the obtained results. Therefore, it is advantageous to complex respective precipitates and preferably separate the complexes from the remaining lysed sample, to provide a cleared lysed sample. Separation can be assisted e.g. by sedimentation or centrifugation. The precipitates may e.g. sediment at the bottom of the tube, leaving behind a supernatant which corresponds to the cleared lysate or the cleared reconstituted composition if said step is performed for that purpose. Sedimentation can be accelerated and improved e.g. by centrifugation. However, it is preferred to assist the separation of the precipitates. Already the complexing of the precipitates has a clearing effect, as contaminants are removed from the remaining sample. However, it is preferred to separate at least a portion of the contaminants from the remaining sample.

According to one aspect, means are provided prior to, during or after lysis for removing precipitates, thereby providing a cleared lysate. The term removing as used in this conjunction means that the amount of precipitates is at least reduced to an extent that the subsequent analytical method can be adequately performed. According to one aspect, at least a portion of the lysis mixture is passed through means that can hold back or remove precipitates during passage of the lysed sample. Suitable means may be selected from the group comprising filter materials, membranes or layers or fillings of particles which bind precipitates. The lysed sample passes through said means and precipitates are caught, respectively are held back by said means thereby providing a cleared sample once the sample has passed the filter. Said means such as e.g. a filter or membrane may be porous. The pores should be sufficiently small to efficiently hold back and thus remove precipitates that are present in the lysis mixture.

According to the first aspect of the present invention, the sample is contacted prior to, during or after lysis with at least one magnetic solid support which binds precipitates, thereby forming a complex with the precipitates. Of course, also more than one complex can be formed, e.g. when using particles such as beads as solid support. Thus, the term "complex" as used herein also refers to a plurality of complexes. Binding to the solid support preferably is unspecific and is achieved by adsorption. The complex is preferably separated from the remaining lysate thereby providing a cleared lysate. Separation of the complex can be assisted by sedimentation, centrifugation or magnetic separation if, as in the first aspect of the invention, a magnetic solid support is used. E.g. the complex can be concentrated at the bottom of the vessel and the supernatant, which corresponds to the cleared lysed sample, can be obtained. Binding the precipitates to the solid support assists the sedimentation of the complex and hence alleviates the removal of the precipitates from the remaining sample. However, the addition of the solid support is also beneficial and provides a clearing effect in that the inhibitory effect of comprised contaminants such as precipitates is reduced, if the complexes are not removed but hence, are present during the analytical method. Apparently, complexing the precipitates by binding them to the solid support already provides a beneficial effect because the bound precipitates are not accessible and hence inactivated.

According to one embodiment, the solid support is selected from the group consisting of particles, plates and other particulate matter. According to one embodiment, the solid support comprises a surface which binds to contaminants, in particular precipitates, that are present in the lysate. The term "binding" is used in a broad sense and refers to any interaction of the contaminants, in particular the precipitates with the solid support that allows to remove at least a portion of said contaminants together with the solid support. Binding preferably is achieved by adsorption.

According to one embodiment, mineral particles comprising or consisting of metal oxides are used as solid support for clearing the lysis mixture. Examples of suitable solid supports include but are not limited to solid supports having a silica surface such as e.g. silica particles or glass particles and polymeric supports. According to one embodiment, the solid support has a hydrous siliceous oxide adsorptive surface. However, the solid support may also comprise one or more ligands to provide a suitable surface for binding the precipitate or to improve binding of the precipitates. According to one embodiment, the ligands are selected from the group consisting of ion exchange groups preferably from the group of cation exchange groups such as carboxyl groups, sulfonate groups and silane ligands. Also combinations of respective ligands can be used. The solid support may additionally or alternatively comprise ligands or compounds on its surface which bind inhibitors of the downstream analysis method, e.g. inhibitors of an amplification reaction. Suitable examples are described above. Preferably, the solid support has a surface comprising carboxyl groups. Exemplary solid supports providing a surface which are suitable for removing precipitates from the lysed sample include small spheres, also known as beads or particles, for example made of glass, silica, polymers or coated materials. In the method of present claim 1, the solid support such as the particles is magnetic. However, it is also possible to modify e.g. the inner surface of the consumable that is used to receive the sample, such as, for example, a vessel. If at least a portion of the inner wall of the vessel that is in contact with the sample comprises respective ligands, the precipitates can bind, preferably adsorb, to said surface and are thereby immobilized to the sample wall.

According to one embodiment the surface of the solid support comprises acidic functional groups. According to one embodiment, the pKa value of said functional groups lies in a range of 0 to 7, preferably 1 to 6, more preferred 1 to 5. Suitable examples include acidic groups such as carboxyl groups. Further suitable functional groups include betains, sulfonate, phosphonate, phenol and phosphate groups. Also mixtures of respective functional groups can be present on the surface. Preferably, the surface comprises carboxyl groups as functional groups, as a respective polyanionic surface is highly efficient in binding and thus depleting precipitates that are formed during lysis thereby additionally providing a lysate clearing effect. The manufacture of carboxylated polymers is well known from the prior art as these polymers are employed in a great number of other technical applications, they are for the most part commercially available. Furthermore, it is also well known how to provide and e.g. functionalize a solid support with a respective polyanionic surface, preferably a carboxylated surface. Examples include but are not limited to coatings, the deposition of a film or layer and the manufacture of the solid support by polyanionic polymers.

Binding of contaminants such as in particular precipitates to the solid support, respectively the surface provided for binding said contaminants, preferably occurs under conditions wherein the contaminants bind to the solid support but wherein the nucleic acids of interest do not or bind to a lesser extent than the biomolecule of interest. Hence, the used lysis conditions are selective in that predominantly the contaminants such as in particular precipitates bind, preferably adsorb, to the solid support, respectively the surface, but wherein there is no substantial binding of the nucleic acids of interest occur so that they remain in a sufficient amount in the lysed sample or in the reconstituted composition to allow performing the intended analytical method.

The solid support for binding the precipitates may be comprised in the lysis solution. Thereby, it is ensured that any precipitates that are formed upon lysis of the sample are directly bound to the solid support upon their formation, thereby forming a complex. Thereby, precipitates are depleted from the remaining sample. Performing the lysis in the presence of the solid support that is used for removing contaminants such as precipitates improves the achieved clearing result. This probably as precipitates can be directly bound while they are being formed. This irrespective of whether lysis is achieved prior to reconstitution of the dry composition or afterwards. Clearing of the lysis mixture may further improve the results of the analytical method, in particular the amplification reaction.

According to one embodiment, the solid support comprises or is a molecular sieve. A molecular sieve is a material containing small pores of a precise and uniform size that may be used as an adsorbent. Preferably, the molecular sieve comprises aluminosilicate minerals, clays, porous glasses, microporous charcoals, zeolites, active carbons, or synthetic compounds that have open structures through which small molecules, such as water can diffuse. Molecular sieves such as zeolites are particularly effective in removing contaminants such as precipitates from a lysed sample. The molecular sieve may be added to the lysis composition which preferably is a lysis solution. They may also be used in addition to the solid supports described above. When processing acidic samples, such as vaginal swab samples using a molecular sieve such as a zeolite as solid support has the specific advantage that zeolites are capable of elevating the pH value of the lysis mixture. This is beneficial as many analysis methods such as e.g. amplification reactions do not work properly at acidic pH values. Hence, according to one embodiment, at least one type of solid support is added prior, during or after lysis which has the effect that the pH value of the lysed sample is elevated. This embodiment is particularly suitable when processing acidic samples having a pH below 7, below 6.5, below 6, below 5.5, below 5 or below 4.5. Preferably, a molecular sieve, more preferred zeolites, are used for this purpose. The molecular sieve can also be added in addition to other solid supports that bind precipitates such as carboxylated particles.

However, as is shown by the presented examples, a respective clearing step is not necessarily required. The obtained lysis mixture, which is optionally further processed such as cleared, can then be used directly in a nucleic acid analysis method, preferably in a nucleic acid amplification reaction. If the nucleic acid of interest is RNA, it is preferred to first perform a reverse transcription step to generate cDNA prior to performing the amplification. The lysate that is obtained with the present invention allows a reverse transcription directly on the obtained lysate. There is no necessity to isolate the RNA in advance. Furthermore, a DNase digest (if the target nucleic acid is RNA) or an RNase digest (if the target nucleic acid is DNA) may be performed prior to amplification. The obtained lysate is also suitable for these purposes. Respectively processed lysates are also encompassed by the term lysate as used herein.

For example, an aliquot of the obtained lysis mixture, which is optionally cleared, can be contacted with reagents that are required for performing an amplification reaction. According to one embodiment, the lysis mixture, which is optionally cleared, is added to an aqueous solution comprising reagents that are necessary for performing an amplification reaction. The amount of used lysis mixture, which is optionally cleared, that is added to the composition comprising the reagents for performing the analysis method such as e.g. the amplification reaction and hence is comprised in the resulting mixture can largely vary and can be chosen e.g. from 1% to 98%, 1% to 95%, 5% to 85%, 10% to 75% and 15% to 70%. Hence, the lysis mixture, which is optionally cleared, can be used in small amounts as well as in rather large amounts in the analysis reaction such as the amplification reaction. The teachings of the present invention in particular have a positive effect if high amounts of lysis mixture, which is optionally cleared, are added, because the inhibition, respectively disturbance of the reaction, which results from the inhibitors comprised in the lysis mixture is advantageously reduced as is demonstrated by the examples.

Furthermore, a lysis mixture that was obtained using the teachings of the present invention can also be used to reconstitute a dry composition, preferably a freeze-dried composition, comprising reagents for performing an analytical reaction such as an amplification reaction. Also in this case, at least a portion of the lysis mixture is used in a nucleic acid analysis method in the sense of the present invention. The dry composition may comprise one or more reagents necessary for performing the intended analytical method such as an amplification reaction. Preferably, the dry composition comprises at least one polymerase. For analysing RNA, the dry composition may comprise a reverse transcriptase. According to one embodiment, the dry composition is a freeze-dried composition. Freeze-dried compositions are widely used for providing reagents necessary for amplification reactions in a storable form. Methods for preparing respective freeze-dried compositions as well as suitable additives that stabilise the comprised reaction compositions, in particular biochemical components such as proteins are well-known in the prior art (see e.g. Freeze-Drying/Lyophilization of Pharmaceutical and Biological Products, Second Edition, WO 01/92569, WO 2010/001162, US 2010/0068716 and US 2010/0159529) and thus, need no detailed description here. The dry composition that can be used in the method according to the present invention is a storable composition. Preferably, it is suitable for long-term storage. According to one embodiment, the dry composition is stable during storage for a time period of at least 3 months, at least 6 months, at least 10 months or at least 12 months. Preferably, the dry composition is stable for a time period of 3 to 18 months or 6 to 12 months. According to one embodiment, the dry composition comprises at least some of the chemical and/or biochemical reagents necessary for conducting the intended analysis method. Preferably, it comprises all of the necessary reagents because upon addition of the lysate the composition is ready for performing the analytical method. This is particularly advantageous when using the method e.g. in a LoC system. According to a preferred embodiment, the dry composition comprises at least some, preferably all, of the reagents necessary for conducting an amplification reaction, preferably a PCR reaction. As discussed above, respective dry compositions, in particular freeze-dried compositions, are widely used to provide the reagents necessary for an amplification reaction in form of a so-called master mix, in a storable form. When intending to perform the amplification reaction, the dry composition only needs to be reconstituted using the lysis mixture to form the amplification reaction mixture, wherein, however, optionally further reagents can be added e.g. if not all reagents were already comprised in the dry composition, what is, however, preferred. Preferably, the dry composition comprises one or more, preferably all reagents selected from the group consisting of a polymerase, a reaction buffer suitable for performing an amplification reaction and dNTPs. Preferably, it also comprises primers and/or labelled probes which allow e.g. the detection the presence or absence of one or more target nucleic acids that are, e.g., indicative of a certain disease or infection. However, primers and/or probes may also be added separately. Furthermore, an internatl amplification control may be comprised. After reconstitution using the lysis mixture and optionally the addition of further additives, such as primers and/or probes, the resulting reconstituted composition is ready for performing the amplification of a target nucleic acid. According to one embodiment, at least 60%, at least 70%, at least 80%, at least 90%, at least 95% or 100% of the liquid that is used for reconstitution is provided by the lysis mixture. Reconstitution can be advantageously achieved exclusively by addition of the lysis mixture. According to one embodiment, at least an aliquot of the lysis mixture, which optionally is further processed such as cleared in advance, that was obtained as described above, is contacted with the dry composition for reconstitution. In order to ensure that the reconstituted composition comprises the reagents in a concentration appropriate for the intended analysis method, a predetermined amount of lysis mixture is added to the dry composition. Said predetermined amount is chosen such so that in the reconstituted composition, the reagents are comprised therein in a concentration suitable for performing the amplification reaction. The reconstitution process can be assisted by agitation e.g. by pipetting the resulting mixture up and down, stirring, shaking or vortexing.

According to one embodiment, the lysis mixture is not cleared prior to performing a nucleic acid amplification method.

The subsequent analysis of the nucleic acids can be performed using any nucleic acid analysis method including, but not limited to, identification technologies, amplification technologies, polymerase chain reaction (PCR), isothermal amplification, reverse transcription polymerase chain reaction (RT-PCR), reverse transcription, reverse transcription amplification, quantitative real time polymerase chain reaction (qPCR), fluorescence detection, hybridization assays, DNA or RNA sequencing, restriction analysis, reverse transcription, NASBA, LAMP (loop mediated isothermal amplification), RPA (recombinase polymerase amplification), tHDA (helix dependent amplification), NEAR (nicking enzyme amplification reaction), TMA (transcription mediated amplification) and NASBA (nucleic acid sequence based amplification), allele specific polymerase chain reaction, polymerase cycling assembly (PCA), asymmetric polymerase chain reaction, linear after the exponential polymerase chain reaction (LATE-PCR), helicase-dependent amplification (HDA), hot-start polymerase chain reaction, intersequence-specific polymerase chain reaction (ISSR), inverse polymerase chain reaction, ligation mediated polymerase chain reaction, methylation specific polymerase chain reaction (MSP), multiplex polymerase chain reaction, nested polymerase chain reaction, solid phase polymerase chain reaction, detection technologies using labeled, preferably fluorescently labeled primers and/or probes, or any combination of the foregoing. Respective technologies are well-known to the skilled person and, thus, do not need further description here. Preferably, the analytical method is a nucleic acid amplification method.

The use of at least one water-soluble polyanionic polymer comprising carboxylate-containing monomers for depleting inhibitors that are released during lysis of the sample has important advantages over prior art methods. As discussed, compared to binders/thickeners such as PVP that were previously used in the prior art, the method according to the present invention is more efficient as is also shown by the examples. Furthermore, the present inventors found that water-soluble polyanionic polymers such as PAA may inhibit an amplification reaction if no sufficient biological sample material was added during lysis, but do not inhibit the amplification reaction if sufficient biological sample material was added during lysis and in this case, as is shown by the exampels, improve the amplification performance by depleting inhibitors that are released during lysis of the sample. Without wishing to be bound by theory it is believed that if no sufficient sample material is added for lysis no or not sufficient inhibitors are released from the sample to "neutralize" or otherwise counteract the amplification inhibitory action of water-soluble polyanionic polymers such as PAA. This advantageous characteristic allows to control that a valid amount of sample material was included in the amplification assay and accordingly allows to determine that the assay result is reliable. Thereby, an integrated control can be provided that allows to identify false negative assay results that may occur if no or an insufficient amount of biological sample material was added for lysis. Thereby, the safety of amplification based analytical assays, in particular diagnostic assays, can be improved. For this purpose of an integrated control, the concentration of the water-soluble polyanionic polymer in the lysis mixture is chosen such that when the desired amount of lysis mixture is transferred into the amplification reaction, the co-transferred water-soluble polyanionic polymer inhibits the amplification reaction if no sufficient sample material was added for obtaining the lysate while it does not inhibit the amplification reaction if sufficient biological sample material was added for obtaining the lysate. The suitable concentration for the individual case can be determined by routine experiments and depends in particular on the type of sample material, the amount of sample material to be processed, the amount of lysis mixture that is subjected to the amplification reaction and the used polymer. According to one embodiment, the amplification reaction comprises an amplification control. Here, internal controls that are commonly used in the prior art may be used. The internal control may be a nucleic acid such as DNA or RNA. If the target nucleic acid to be amplified is RNA, the control preferably also is provided by RNA. The amplification reaction comprises suitable primers and/or probes that allow to detect the internal control under the used amplification conditions. In conjunction with the embodiment wherein the inhibitory water-soluble polyanionic polymers such as PAA are used to control that sufficient sample material was added for lysis, said amplification control only provides an amplification signal if the biological sample was added for obtaining the lysate. If no or not enough sample material was added for lysis, no amplification signal is obtained for the amplification control because the inhibitory water-soluble polyanionic polymer, e.g. PAA, present in the amplification reaction then inhibits the amplification. This indicates that the diagnostic assay did not work properly. Thereby also amplification reactions can be identified as being invalid wherein no sufficient biological sample material was added. Therefore, incorporating a respective internal control has advantages. To efficiently deplete inhibitors while at the same time providing the option for an integrated control that is provided when using inhibitory water-soluble polyanionic polymers such as PAA as is taught by the present invention is not provided when using e.g. PVP for depleting inhibitors.

The term "sample" is used herein in a broad sense and is intended to include sources that contain biomolecules, in particular nucleic acids and proteins. Exemplary samples include, but are not limited to, biological samples such as body fluids in general, whole blood, serum, plasma, red blood cells, white blood cells, buffy coat; swabs, including but not limited to buccal swabs, throat swabs, vaginal swabs, urethral swabs, cervical swabs, throat swabs, rectal swabs, lesion swabs, abcess swabs, nasopharyngeal swabs and anal swabs, urine, sputum, saliva, semen, lymphatic fluid, liquor, amniotic fluid, cerebrospinal fluid, peritoneal effusions, feces, pleural effusions, fluid from cysts, synovial fluid, vitreous humor; aqueous humor, bursa fluid, eye washes, eye aspirates, pulmonary lavage, lung aspirates, tissues, including but not limited to, liver, spleen, kidney, lung, intestine, brain, heart, muscle, pancreas and cell cultures, bacteria, microorganisms, viruses, plants, fungi including samples that derive from the foregoing or comprise the foregoing. Materials obtained from clinical or forensic settings or enviromental samples such as soil that contain or are suspected to contain nucleic acids are also within the intended meaning of the term sample. Furthermore, the skilled artisan will appreciate that extracts, or materials or portions thereof obtained from any of the above exemplary samples are also within the scope of the term sample. Preferably, the sample is derived from a human, animal, plant, bacteria or fungi. Preferably, the sample is selected from the group consisting of cells, tissue, bacteria, viruses and body fluids such as for example blood, blood products such as buffy coat, plasma and serum, urine, liquor, sputum, stool, CSF and sperm, epithelial swabs, vaginal swabs, cervix samples, biopsies, bone marrow samples and tissue samples, preferably organ tissue samples such as lung and liver. The sample may be stabilized. Certain samples such as blood samples are usually stabilised upon collection, e.g. by contacting them with a stabilizer such as an anticoagulant in case of blood and samples derived from blood.

Furthermore, the method according to the present invention can advantageously be used to lyse a fixed sample. According to this embodiment, a method is provided for lysing a fixed sample to provide a lysis mixture that is suitable for being directly used in a nucleic acid analysis method, wherein the fixed sample is contacted with
(a) at least one non-ionic surfactant,
(b) at least one water-soluble polyanionic polymer **comprising carboxylate-containing monomers ,** wherein said polymer is (i) a polymer consisting of carboxylate-containing monomers that are selected from acrylic acid, methacrylic acid and/or maleic acid, or
   (ii) a polymer consisting of carboxylate-containing monomers that are selected from acrylic acid, methacrylic acid and/or maleic acid and additional polar monomers without a carboxylate function that are selected from lactide and/or vinylpyrrolidone,
   optionally wherein said polymer is a polymer selected from polyacrylic acid, polymaleic acid, polymethacrylic acid, poly(acrylic acid-co-maleic acid), poly(acrylic acid-co-lactide), poly(acrylic acid-co-vinylpyrrolidone), poly(maleic acid-co-lactide), poly(maleic acid-co-vinylpyrrolidone), poly(acrylic acid-co-maleic acid-co-lactide) and poly(acrylic acid-co-maleic acid-co-vinylpyrrolidone),
   wherein preferably polyacrylic acid is used,
and heated at ≥ 85°C, preferably ≥ 90°C, to provide a lysis mixture.

As is shown by the examples, the lysis mixture that is respectively obtained from the fixed sample is directly suitable for use in a nucleic acid analysis method such as an amplification reaction. In contrast to prior art methods, no nucleic acid isolation is necessary. Said method is rapid, automatable and enables high throughput processing of large amounts of fixed samples such as e.g. FFPE samples present in biobanks or other clinical settings. Therefore, the method according to the present invention has particular advantages in this specific field. Including a heating step is important when processing fixed samples to ensure an efficient release of the comprised nucleic acids. Furthermore, performing a respective heating step during lysis has the advantage that nucleases comprised in the lysis mixture are denatured and thereby are inactivated. The necessary heating incubation time depends on the type of fixation, the degree of fixation, the amount of fixed sample to be lysed and the incubation temperature. Preferably, heating is performed at ≥ 90°C, preferably ≥ 95°C. Such heating step is also referred to as boiling lysis. Preferably, heating is performed for at least 5min, at least 7.5min, at least 10min, at least 12.5min, at least 15min, at least 17.5 min, at least 20min, at least 22.5min, at least 25min, at least 27.5min or at least 30min. Suitable heating periods may be selected from 5min to 45min, 7.5min to 42.5min, 10min to 40min, 12.5min to 37.5min and 15min to 35min. As is shown in the examples, said method allows to lyse the fixed biological sample and release the comprised nucleic acids from fixed samples thereby rendering the nucleic acids accessible to analytical methods such as reverse transcription and/or amplification. Furthermore, as it is ensured due to the presence of the at least one water-soluble polyanionic polymer that potential inhibitors of the analytical method are efficiently depleted, the obtained lysate can be directly used in the nucleic acid analysis method such as e.g. an amplification reaction. The inhibitor depletion performance is also in conjunction with fixed samples superior to prior art depletion methods.

Preferably, after the heat incubation step, the obtained lysis mixture is mixed, e. g. vortexed or otherwise strongly agitated or shaked. Furthermore, the heated lysis mixture is preferably allowed to cool down. It may be cooled at room temperature or below. Preferably, it is allowed to cool down to a temperature below 50°C, more preferably it is cooled down at least down to room temperature. Such a cooling step is advantageous in particular when processing embedded fixed samples such as paraffin embedded fixed samples because the embedding material solidifies during cooling thereby facilitating to prevent a carry-over of embedding material. Furthermore, it was found that a respective cooling step promotes the formation of precipitates which comprise, respectively constitute themselves inhibitors or contaminants of the subsequent analytical method. The efficient precipitate formation allows to efficiently remove respective precipitates e.g. by performing a clearing step as is described herein, thereby providing a lysate from which inhibitors and contaminants such as precipitates are efficiently depleted. Suitable and preferred embodiments of a respective clearing step are described herein. Preferably, a solid support comprising anionic groups such as in partiuclar carboxyl groups at its surface is used for clearing. It is preferred to provide the solid support suitable for capturing precipitates that are formed during lysis of the fixed sample prior to heating. This is particularly feasible if particles are used as solid support. The particles can be e.g. added for lysis. Thereby, it is ensured that precipitates that are formed upon lysis of the fixed biological sample, what in particular occurs during heating, are directly bound to the solid support upon their formation and thus are efficiently depleted from the remaining sample e.g. by separating the solid support with the bound contaminants from the remaining lysis mixture which is thereby cleared.

Preferably, the fixed sample is a tissue sample, preferably a fixed organ tissue sample, most preferred obtained from a human or rodent. The tissue may be obtained from autopsy, biopsy or from surgery. It may be a solid tissue such as, for example, parenchyme, connective or fatty tissue, heart or skeletal muscle, smooth muscle, skin, brain, kidney, liver, spleen, breast, carcinoma (e.g. bowel, nasopharynx, breast, lung, stomach etc.), cartilage, lymphoma, meningioma, placenta, prostate, thymus, tonsil, umbilical cord or uterus. The tissue may be a tumor (benign or malignant), cancerous or precancerous, obtained from an animal or human subject affected by disease or suspected of same (normal or diseased), or be affected by other pathology. It may be obtained by autopsy, biopsy (e. g., endoscopy or laparoscopy), or surgical resection. Basically any sample that can be fixed using common prior art methods can be processed. Fixation of the biological sample can be effected with any fixative known to the person skilled in the art, in particular with acids, alcohols, ketones or other organic substances, such as, in particular, glutaraldehyde, formaldehyde or paraformaldehyde. Examples of fixatives and uses thereof may be found in Sambrook et al. (2000); Maniatis et al. (1989). Preferably, the used fixation also preserves DNA and RNA. According to one embodiment, the used fixation stabilizes the morphology of the sample also at ambient temperatures at least for several weeks or months. According to one embodiment, the fixative has a crosslinking effect such as formaldehyde and paraformaldehyde. Biological samples fixed with formaldehyde can be efficiently processed and thus lysed with the method according to the present invention. According to one embodiment of the process according to the invention, a formaldehyde-fixed, paraffin-embedded biological sample (FFPE sample) is used. Other fixatives and fixation methods for providing a fixed biological sample are known in the prior art. In particular alcohol based fixed samples can be used. A respective fixation process involving alcohols is e.g. described in WO 2008/104564. As is shown by the examples, samples fixed according to said procedure can be efficiently lysed with the method according to the present invention and the obtained lysate can be used directly in downstream analytical methods such as amplification reactions. Furthermore, the fixed tissue may or may not be embedded in a non-reactive substance such as paraffin. Embedding materials include but are not limited to paraffin, mineral oil, non- water soluble waxes, celloidin, polyethylene glycols, polyvinyl alcohol, agar, gelatine, nitrocelluloses, methacrylate resins, epoxy resins or other plastic media. Thereby, one can easily produce tissue sections of the biological material suitable for histological examinations. Said embedding steps preferably are carried out under mild conditions, e.g. with low melting paraffin (wax). Said steps can be carried out manually or by any automated system. Methods and compositions of the invention can be applied to any fixed cell or tissue sample, whether it has been embedded or not. According to one embodiment, the biological sample is fixed with formalin, in particular after the sample has been embedded in a non-reactive substance such as paraffin. Tissue sections can then be prepared from the paraffin embedded sample by means of suitable cutting devices, for example by means of a microtome, the thickness of these sections normally being about 5 to 20 µm for examination under a light microscope. Furthermore the paraffin-embedded sample can also be reduced in size by other methods, for instance by perforating with a hollow needle or by the so-called laser capture method, to give smaller sample fragments.

When processing fixed samples that have been embedded in an embedding material such as paraffin, it is within the scope of the present to include a step to remove said embedding material prior to lysis. Thus, according to one embodiment wherein the fixed sample is a sample embedded in an embedding material such as paraffin, the embedding material is at least partly, preferably completely, removed from the fixed biological sample prior to lysis. The removal of the embedding material such as paraffin from the biological sample can in principle take place by all methods known to the skilled worker for deparaffinization of biological samples. The deparaffinization preferably takes place by initially contacting the sample with a hydrophobic organic solvent, in particular with an aromatic hydrocarbon, such as xylene, in order to dissolve out the embedding material such as paraffin. Suitable deparaffinization methods are e.g. described in WO 2011/104027, WO 2011/157683 and WO 2007/068764. After removal of the embedding material the sample it may be rehydrated, this rehydration may e.g. take place by stepwise washing with aqueous alcohol solutions with decreasing alcohol concentration (=descending alcohol series). However, as is shown by the examples, it is not required to remove the non-reactive embedding material such as paraffin prior to lysis, if a respectively embedded fixed sample is processed using the method according to the present invention. E.g. during heating, the sample is automatically deparaffinized as the paraffin melts. The paraffin forms a layer or small chunks on the lysis mixtue and resolidifies upon cooling of the lysis mixture. The lysis mixture - which is free of paraffin - can then be obtained e.g. by pipetting through said paraffin layer. It is a great advantage that it is not necessary to include a separate embedding material removal step as this saves time and chemistry and contributes to that the method can be performed very rapidly and cost-efficient and furthermore, is suitable for automation. The same applies to other embedding materials besides paraffin which melt under the heating conditions used.

The method according to the present invention for lysing a fixed sample does not require a step wherein the nucleic acids are isolated from the lysed sample e.g. by binding them to a solid support and/or by precipitating them out of the lysis mixture in order to purify them prior to using them in an analytical method. Rather, inhibitors that could interfere with the intended analysis method and preferably also precipitates are depleted as described above using the water-soluble polyanionic polymer preferably comprising carboxylate-containing monomers. This ensures that the analysis method can be efficiently performed even if a large amount of the obtained lysis mixture, which preferably is cleared, is used. Hence, e.g. the analysis method may be carried out in immediate succession after lysis using an aliquot of the obtained lysis mixture which optionally but preferably is cleared, without the need for performing a purification of the nucleic acids between lysis and analytical method. This makes the method rapid and particularly suitable for automation.

Furthermore, said method for lysing fixed samples does in contrast to prior art methods not require the use of a proteolytic enzyme such as e.g. subtilisins, proteinase K, trypsin and other enzymes. This is a great advantage, because the use of respective enzymes is costly and furthermore, often prolongs the overall preparation time as the enzymatic digestion step requires longer incubation times at moderately elevated temperatures. The present invention, wherein no proteolytic enzyme is used for lysing the fixed sample, therefore has important advantages. Furthermore, no chaotropic agents such as guanidine salts, thiocyanate salts, isothiocyanate salts, perchlorates, iodide salts and urea are required for lysing the sample and thus are not used during lysis. In fact, the method according to the present invention does not require the use of any chaotropic salts or chaotropic agents. This is a further important advantage, as the method according to the present invention can be carried out without the use of respective toxic agents.

The term "nucleic acid" or "nucleic acids" as used herein, in particular refers to a polymer comprising ribonucleosides and/or deoxyribonucleosides that are covalently bonded, typically by phosphodiester linkages between subunits, but in some cases by phosphorothioates, methylphosphonates, and the like. Nucleic acids include, but are not limited to all types of DNA and/or RNA, e.g. gDNA; circular DNA; plasmid DNA; circulating DNA; PNA; LNA, cyclohexene nucleic acids; RNA/DNA hybrids; hnRNA; mRNA; noncoding RNA (ncRNA), including but not limited to rRNA, tRNA, miRNA (micro RNA), siRNA (small interfering RNA), snoRNA (small nucleolar RNA), snRNA (small nuclear RNA), pwi-interacting RNA (piRNA), repeat associated RNA (rasiRNA), as RNA and stRNA (small temporal RNA); fragmented nucleic acid; nucleic acid obtained from subcellular organelles such as mitochondria or chloroplasts; and nucleic acid obtained from pathogens, microorganisms, parasites, or DNA or RNA viruses that may be present in a biological sample, e.g. bacteria, viral or fungi nucleic acids; synthetic nucleic acids, extracellular nucleic acids. The term "extracellular nucleic acids" or "extracellular nucleic acid" as used herein, in particular refers to nucleic acids that are not contained in cells. Respective extracellular nucleic acids are also often referred to as cell-free nucleic acids. These terms are used as synonyms herein. The term "extracellular nucleic acids" refers e.g. to extracellular RNA as well as to extracellular DNA. Examples of typical extracellular nucleic acids that are found in the cell-free fraction (respectively portion) of biological samples such as body fluids such as e.g. blood plasma include but are not limited to mammalian extracellular nucleic acids such as e.g. extracellular tumor-associated or tumor-derived DNA and/or RNA, other extracellular disease-related DNA and/or RNA, epigenetically modified DNA, fetal DNA and/or RNA, small interfering RNA such as e.g. miRNA and siRNA, and non-mammalian extracellular nucleic acids such as e.g. viral nucleic acids, pathogen nucleic acids released into the extracellular nucleic acid population e.g. from prokaryotes (e.g. bacteria), viruses or fungi.

According to a second aspect, a nucleic acid amplification method is provided, comprising
a) lysing a sample comprising nucleic acids according to the method of the first aspect of the present invention,
b) using at least a portion of the lysis mixture in an amplification reaction.

Details with respect to step a) were described above in conjunction with the method according to the first aspect of the present invention. It is referred to the above disclosure which also applies here.

Suitable amplification reactions that can be performed in step b) are also described above. It is referred to the respective disclosure which also applies here. In a respective amplification step, nucleic acids or specific target nucleic acids comprised in the lysis mixture are multiplied by means of a method which amplifies the nucleic acids. Such methods are well known in expert circles and are not limiting for the invention. A preferred example for such a method is a polymerase chain reaction (PCR), if appropriate with a preceding reverse transcription in case the nucleic acid of interest is RNA. The amplification may be performed with specific primers or with unspecific primers. By using specific primers, it is possible to enhance either one or more specific gene sequence(s) from the genetic material, or, when using specific primers according to international standard, a genetic fingerprint may be generated. In addition, it is also possible to use unspecific primers, e.g. when comparing a known sample with an unknown sample according to the size pattern of the unspecific amplicons in order to find out about the origin of the unknown sample.

As discussed above, due to the incorporation of the water-soluble polyanionic polymer during lysis it is not necessary to isolate and in particular to purify the nucleic acids comprised in the lysis mixture prior to performing the amplification method. Therefore, no nucleic acid isolation method is performed between steps a) and b). However, as described above it is within the scope of the present invention to further process the lysis mixture prior to subjecting an aliquot thereof into the amplification reaction. E.g. the lysis mixture may be cleared and an aliquot of the cleared lysate is then used in the amplification reaction. The clearing may involve e.g. the removal of precipitates. This can be achieved e.g. by sedimenting the precipitates which may be assisted by centrifugation. Alternatively, precipitates may be removed e.g. by using a filter or by adding a solid support that binds precipitates. Details were described above, it is referred to the respective disclosure. According to one embodiment, however, the lysis mixture is not cleared prior to step b). Further intermediate lysis mixture processing steps such as e.g. enzymatic treatments were also described above and respectively treated lysis mixtures are also encompassed by the term lysis mixture.

According to a third aspect, a lysis composition is provided comprising
(a) at least one non-ionic surfactant,
(b) at least one water-soluble polyanionic polymer comprising carboxylate-containing monomers, wherein the at least one water-soluble polyanionic polymer or mixture of water-soluble polyanionic polymers is comprised in the lysis solution in a concentration of 0.01% (w/v) to 1% (w/v), and wherein said polymer is
   (i) a polymer consisting of carboxylate-containing monomers that are selected from acrylic acid, methacrylic acid and/or maleic acid, or
   (ii) a polymer consisting of carboxylate-containing monomers that are selected from acrylic acid, methacrylic acid and/or maleic acid and additional polar monomers without a carboxylate function that are selected from lactide and/or vinylpyrrolidone,
   optionally wherein said polymer is a polymer selected from polyacrylic acid, polymaleic acid, polymethacrylic acid, poly(acrylic acid-co-maleic acid), poly(acrylic acid-co-lactide), poly(acrylic acid-co-vinylpyrrolidone), poly(maleic acid-co-lactide), poly(maleic acid-co-vinylpyrrolidone), poly(acrylic acid-co-maleic acid-co-lactide) and poly(acrylic acid-co-maleic acid-co-vinylpyrrolidone),
and optionally
(c) at least one proteolytic enzyme,
(d) at least one chelating agent for divalent cations, and/or
(e) at least one buffer substance.

Details with respect to each additive (a), (b), (c), (d) and (e), preferred embodiments and suitable concentrations were described above in conjunction with the method according to the first aspect of the present invention. Also the specific advantages were explained. Said lysis composition may be advantageously used to lyse samples, including fixed samples, and provides a lysis mixture that is directly suitable for use in an amplification reaction. It is referred to the above disclosure which also applies here. As discussed above, the lysis composition is preferably a lysis solution. A particularly preferred lysis solution comprises
- as additive (a) a mixture of two non-ionic detergents, preferably selected from polyoxyethylene fatty alcohol ethers, polysorbates and/or polyoxyethylene alkyl phenyl ethers, more preferred from polysorbate 20 (Tween 20) and nonylphenylpolyethylenglycol (Nonidet P40), wherein each non-ionic detergent is comprised in a concentration of 0.2% (v/v) to 0.6% (v/v), preferably 0.4% (v/v) to 0.5% (v/v);
- as additive (b), the water-soluble polyanionic polymer, which preferably is or comprises polyacrylic acid, in a concentration of 0.01% (w/v) to 0.5% (w/v), preferably 0.03% (w/v) to 0.2% (w/v);
- optionally a proteolytic enzyme such as proteinase K as additive (c);
- as additive (d) EDTA in a concentration below 1.5mM;
- as additive (e) a buffer substance, preferably TRIS, in a concentration of 7.5mM to 50mM, preferably 10mM.

Further disclosed herein is a kit comprising a lysis composition according to the third aspect of the present invention. Details with respect to the lysis composition are described above, it is referred to the respective disclosure. Preferably, said kit additionally comprises one or more reagents for performing an amplification reaction. A respective kit simplifies to carry out the lysis and/or the amplification method according to the present invention.

Numeric ranges described herein are inclusive of the numbers defining the range. The headings provided herein are not limitations of the various aspects or embodiments of this invention which can be read by reference to the specification as a whole. The term "carboxylate" as used herein also refers to protonated carboxylate groups, e.g. carboxyl groups, since depending on the pH value of the lysis mixture always a certain amount, preferably a minor amount, of the carboxylate groups is protonated. Thus, the terms "carboxylate" and "carboxyl group" are used interchangeably herein and shall refer to the carboxyl group (COOH), the carboxylate anion (COO⁻) or the carboxylate salt [-COO⁽⁻⁾ X⁽⁺⁾]. Likewise, the term "acid" as used herein also refers to the salts of the respective acid, i.e. to the deprotonated form of the acid and vice versa. Thus, contacting the sample with at least one water-soluble polyanionic polymer e.g. also comprises adding a respective polymer with anionizable groups which becomes a polyanionic polymer under the conditions used during lysis.

Preferably, preferred embodiments as described herein are used in combination with each other. Furthermore, embodiments that are described herein as comprising a certain subject-matter or steps according to one embodiment may also consist or substantially consist of the respective subject-matter or steps.

### FIGURES

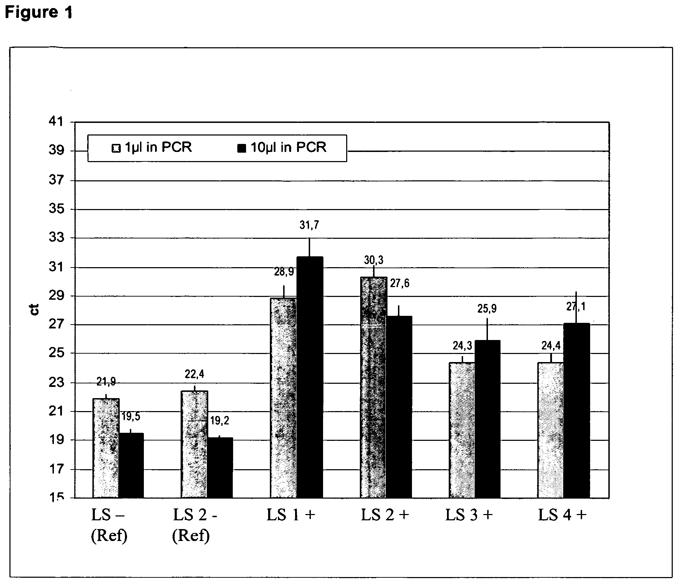

**Figure 1****:** Inhibition of PCR amplification by blood lysates. The Ct-values (with standard deviation) obtained by quantitative real-time-PCR (q-PCR) refer to the upstream ORF of eaeA gene of E. coli 0157:H7. LS 1, LS 2, LS 3, LS 4 = lysis solutions (see example 1), E. coli lysate: - = without, + = with blood lysate. As can be seen from the reduced ct values, the lysis buffers according to the present invention (LS 3 and LS 4) significantly reduce the inhibitory effects of the blood lysate.

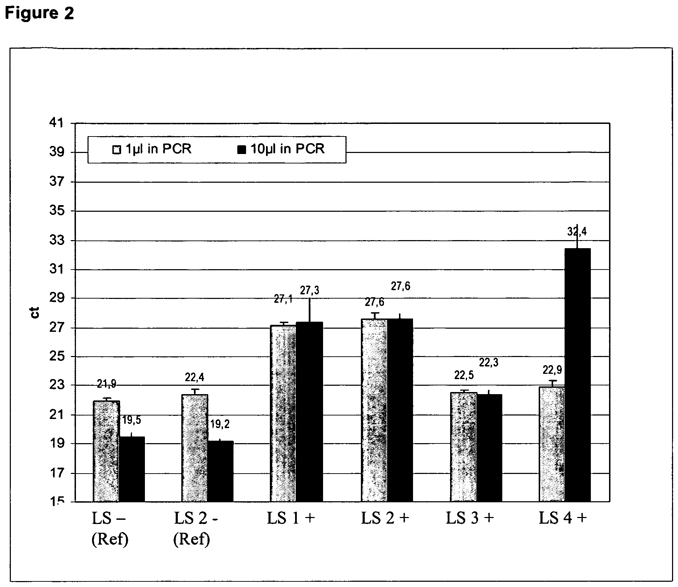

**Figure 2****:** Inhibition of PCR amplification by plasma lysates. The Ct-values (with standard deviation) obtained by q-PCR refer to the upstream ORF of eaeA gene of E. coli. LS 1, LS 2, LS 3, LS 4 = lysis solutions (see example 1), E. coli lysate: - = without, + = with plasma lysate. As can be seen from the reduced ct values, the lysis buffers according to the present invention (LS 3 and LS 4) significantly reduce the inhibitory effects of the plasma lysate.

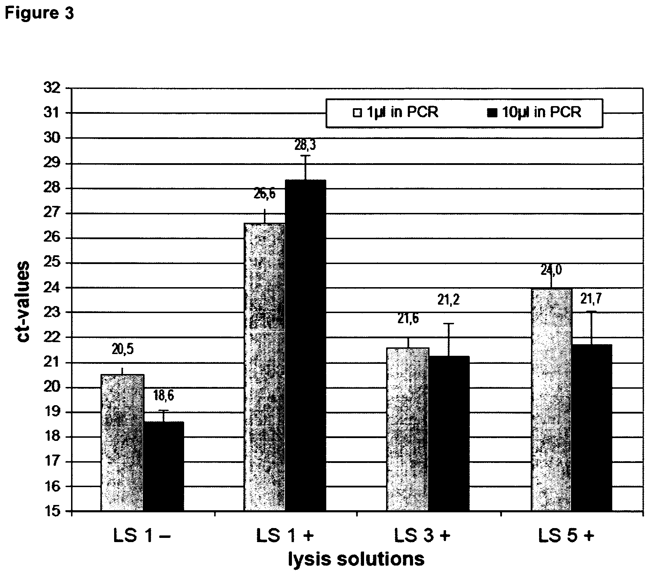

**Figure 3****:** Inhibition of PCR amplification by blood lysates. The Ct-values (with standard deviation) obtained by q-PCR refer to the upstream ORF of eaeA gene of E. coli. 1 µl (grey bar) and 10 µl (black bar) of blood lysate in q-PCR mix. LS 1, LS 3, LS 5 = lysis solutions (see example 2), E. coli lysate: - = without, + = with blood lysate. As can be seen from the reduced ct values, the lysis buffers according to the present invention (LS 3 and LS 5) significantly reduce the inhibitory effects of the blood lysate.

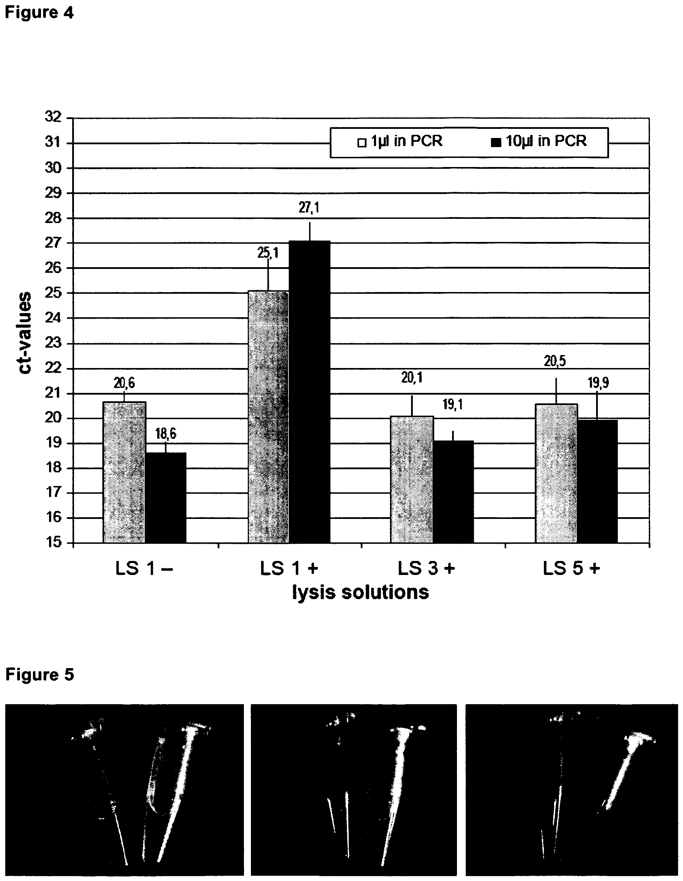

**Figure 4****:** Inhibition of PCR amplification by plasma lysates. The Ct-values (with standard deviation) obtained by q-PCR refer to the upstream ORF of eaeA gene of E. coli. 1 µl (grey bar) and 10 µl (black bar) of blood lysate in q-PCR mix. LS 1, LS 3, LS 5 = lysis solutions (see example 2), E. coli lysate: - = without, + = with plasma lysate. As can be seen from the reduced ct values, the lysis buffers according to the present invention (LS 3 and LS 5) significantly reduce the inhibitory effects of the plasma lysate.

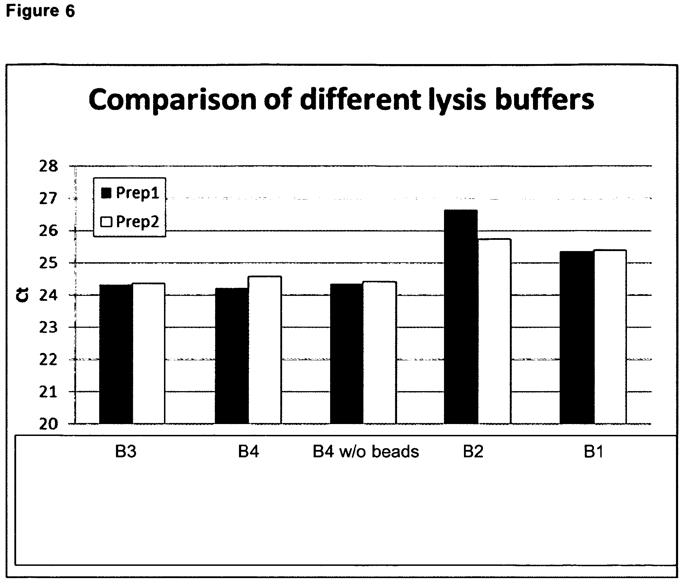

**Figure 5****:** Appearance of blood lysates in comparison. Left: reference without blood; middle: blood lysate not containing additive (b); right: blood lysate containing additive (b). **Figure 6****:** Comparison of the depletion performance of different lysis compositions B1 to B4 in combination with carboxylated beads except for B4 w/o beads, wherein no carboxylated beads were used.

### EXAMPLES

### Example 1: PCR amplification using blood or plasma lysates

The advantageous effects of a water-soluble polyanionic polymer, i.e. additive (b), in a lysis solution of the present invention, on the results of quantitative real-time-PCR (q-PCR) are demonstrated. As sample, blood and plasma were lysed. Both, blood and plasma are known for containing substances which inhibit nucleic acid amplification. Therefore, when adding a lysed sample that was obtained from blood or plasma to a nucleic acid amplification reaction, the amplification reaction becomes increasingly inhibited the more lysate is added to the reaction. Example 1 demonstrates that the amplification can be significantly reduced if a water-soluble polymer is comprised in the lysis solution. Said polymer can compensate PCR inhibitory effects of the lysed sample.

In example 1, four different lysis solutions were tested and compared (LS1 to LS 4). Their composition is shown in the subsequent table. LS 3 and LS 4 are lysis solutions according to the present invention which comprise a water-soluble polyanionic polymer.

| **Lysis buffer** | **Non-ionic detergent** | **Polymer** | **Buffer** | **Chelating agent** | **pH** |
|---|---|---|---|---|---|
| LS 1 | 0,45% Tween | 0,1% PVP | 10mM Tris | 1 mM EDTA | 8,0 |
| | 20 | (10.000 | | | |
| | 0,45% NP40 | MW) | | | |
| LS 2 | 0,45% Tween 20 | NONE | 10mM Tris | 1 mM EDTA | 8,0 |
| | 0,45% NP40 | | | | |
| **LS 3 (invention)** | **0,45% Tween 20** | **0,05% PAA1** | **10mM Tris** | **1mM EDTA** | **8,0** |
| | **0,45% NP40** | | | | |
| **LS 4 (invention)** | **0,45% Tween 20** | **0,1% PAAMA** | **10mM Tris** | **1mM EDTA** | **8,0** |
| | **0,45% NP40** | | | | |

| | | | | | |
|---|---|---|---|---|---|
| PAA1: polyacrylic acid, MW approx. 250,000 Da CAS 9003-01-4, Sigma Aldrich: 416002-250ml PAAMA: poly(acrylic acid-co-maleic acid) sodium salt, MW approx. 50,000 Da CAS: 52255-49-9, SigmaAldrich: 416061-250g. | | | | | |

### a) Blood lysis

For obtaining blood lysates the following additives were mixed:
- 985 µl of LS 1, LS 2, LS 3 or LS 4,
- 5 µl of human blood and
- 10 µl of E. coli cell suspension (= 3 x 10⁶ cells), ATT 700728.

The resulting mixture was incubated for 5min at 95°C under constant shaking at 1.400 rpm on an Eppendorf Thermomixer. The obtained lysis mixture was cooled down to room temperature. 1µl and 10µl supernatant were subsequently used in a 25µl qPCR reaction.

### b) Plasma lysis

For obtaining plasma lysates the following additives were mixed:
- 985 µl of LS 1, LS 2, LS 3 or LS 4,
- 70 µl of human plasma
- 10 µl of E. coli cell suspension (= 3 x 10⁶ cells); ATT 700728.

The resulting mixture was incubated for 5min at 95°C under constant shaking at 1.400 rpm on an Eppendorf Thermomixer. The obtained lysis mixture was cooled down to room temperature. 1µl and 10µl supernatant were subsequently used in a 25µl qPCR reaction.

### c) Reference lysis

As reference, the E.coli bacteria were lysed using buffers LS 1 and LS 2 without the addition of a further sample, i.e. no blood or plasma was added. Thus, the resulting lysis mixture did not comprise or comprised to a much less extent inhibitory additives that originate from the actual sample. The sample was then incubated for 5 min at 95 °C under constant shaking at 1.400 rpm. 1µl and 10µl supernatant were subsequently used in a 25µl qPCR reaction.

### d) Quantitative real Time PCR

Q-PCR was performed for the amplification of an upstream ORF of eaeA gene of E. coli. Thus, primers for the apathogenic E. coli strain 0157:H7 (ATTC 700728) were used:
EFwd: CGA TGA TGC TAC CCC TGA AAA ACT
ERev: TAT TGT CGC TTG AAC TGA TTT CCT C
EPro: "6-Fam"-CGT TGT TAA GTC AAT GGA AAA ACC TG-BHQ1

The 25 µl q-PCR mixes contained the following:

| | |
|---|---|
| QIAGEN QuantiFast Probe PCR Mastermix | 12.50 µl |
| EFwd (100 µM) | 0.10 µl |
| ERev (100 µM) | 0.10 µl |
| EPro (100 µM) | 0.05 µl |
| bidest. water | 11.25 µl |
| lysate aliquots | 1.00 µl or 10 µl |

Temperature cycles were: 5 min at 95 °C and 40 x (15 s at 95 °C, 1 min at 60 °C). The amplification was carried out in each case with 4 lysates prepared seperately.

### Results:

Comparing the quantitative PCR threshold cycle (Ct) values of the lysates that were obtained with the lysis solutions LS 1 and LS 2 with and without the addition of the blood sample, it becomes apparent that compared with the reference method, a difference in the Ct values (delta Ct value) of approximately 7 when using 1 µl lysate per reaction and between 8 and 12 when using 10 µl lysate per reaction is observed. This demonstrates that the lysis in the presence of blood considerably impairs the PCR reaction due to the inhibitors that are comprised in the blood sample and that are released during lysis. Because in a PCR the DNA is amplified in an exponential way, a delta Ct value of 7 corresponds to a factor of 128 and a delta Ct value of 8 corresponds to a factor of 256. Therefore, the blood additives that are released during lysis have a considerable inhibitory effect on the PCR amplification.

As can be derived from Fig. 1, when using the lysis buffers LS 3 and LS 4 according to the present invention, the achieved Ct values are considerably lower than the Ct values that are achieved with the prior art lysis buffers LS 1 and LS 2. For example, when using 1 µl lysis mixture in the PCR, LS 3 achieves at Ct value that is 4 Ct values lower than the Ct value that is achieved with lysis buffer LS 1 and even 6 Ct values lower when compared with the lysis buffer LS 2. When looking at the use of 10 µl lysis mixture in the PCR reaction, the lysis buffer LS 3 achieves a Ct value which is approximately 6 Ct values lower compared to the use of the lysis buffer LS 1 and still approximately 1,7 Ct values lower than the results obtained with LS 2. With respect to lysis buffer LS 4, similar results are obtained. This shows that the method according to the present invention is significantly better in reducing the amount of PCR inhibitors, respectively ameliorating the inhibitory effects of compounds comprised in the lysis mixture.

In case of plasma, the obtained Ct-values are shown in Fig. 2. Comparing the Ct-values of the samples lysed with LS 1 and LS 2 without (-) and with (+) the addition of blood, the Ct-values are increased about approx. 5 in the presence of blood. This delta Ct of 5 means a factor of 32 with respect to the inhibitory effect of plasma on q-PCR. Thus, the PCR is considerably inhibited due to additives that are released from the plasma during lysis.

Comparing the Ct-values of the samples lysed with LS 3 and LS 4 and those of the samples obtained by lysis with LS 1 and LS 2, each in the presence of plasma (+), the Ct-values are decreased by about 5 and 4.5 Ct values in case of LS 3 resp. LS 4. These delta Cts of 5 and 4.5 mean a factor of 32 resp. 23 with respect to the reduction of the inhibitory effect. However, with LS 4 the effects were not improved when adding µl lysis mixture to the PCR reaction. A benefit was only seen when adding 1µl to the PCR reaction. Therefore, the use of polyacrylic acid as water-soluble polyanionic polymer (as in LS 3) is preferred.

To sum it up, using a lysis solution comprising a water-soluble polyanionic polymer, results in a pronounced reduction of the inhibitory effect of blood or plasma on the PCR amplification reaction.

### Example 2: PCR amplification using blood or plasma lysates

Lysis of E. coli cells with or without simultaneous addition of blood or plasma as well as the subsequent q-PCR were carried out according to the procedure described in example 1.

LS 2, LS 3 and another lysis solution (LS 5) according to the present invention were tested. The latter, LS 5, contained 0.05 % polyacrylic acid (MW approx. 100,000 Da) and, furthermore, 0.45 % Tween 20, 0.45 % Nonidet P-40, 10 mM Tris/Cl pH 8.0, 1 mM EDTA. In addition to 1 µl blood or plasma lysate aliquots, 10 µl aliquots were employed (11.25 or 2.25 µl of bidest. water correspondingly) in the 25µl q-PCR mix.

### Results:

In case of blood, the obtained Ct-values are shown in Fig. 3. Comparing the Ct-values of the samples lysed with LS 1 - not containing a additive (b) - without (-) and with (+) the addition of 1 or 10 µl of blood, the Ct-values are increased about approx. 6 resp. 10 in the presence of blood. These delta Cts of 6 and 10 mean a factor of 64 resp. 1024 with respect to the inhibitory effect of blood on PCR.

Comparing the Ct-values of the samples lysed with LS 3 - containing a additive (b) - and those of the samples obtained with LS 1 - not containing a additive (b) -, in the presence of 1 or 10 µl of blood (+), the Ct-values are decreased by about 5 resp. 7 in case of LS 3. These delta Cts of 5 and 7 mean a factor of 32 resp. 128 with respect to the reduction of the inhibitory effect. In case of LS 5 the obtained delta Cts of approx. 2.5 and 6.5 mean a factor of 6 resp. 90 with respect to the reduction of the inhibitory effect.

In case of plasma, the obtained Ct-values are shown in Fig. 4. Comparing the Ct-values of the samples lysed with LS 1 without (-) and with (+) the addition of 1 or 10 µl of plasma, the Ct-values are increased about 4.5 resp. 8.5 in the presence of blood. These delta Cts of 4.5 and 8.5 mean a factor of 23 resp. 360 with respect to the inhibitory effect of plasma on PCR. Comparing the Ct-values of the samples lysed with LS 3 - containing a additive (b) - and those of the samples obtained with LS 1 - not containing a additive (b) -, each in the presence of 1 or 10 µl of plasma (+), the ct-values are decreased by about 5 resp. 8 in case of LS 3. These delta Cts of 5 and 8 mean a factor of 32 resp. 256 with respect to the attenuation of the inhibitory effect. In case of LS 5 the obtained delta Cts of approx. 4.5 and 7 mean a factor of 23 and 128 with respect to the reduction of the inhibitory effect.

To sum it up, using a lysis solution comprising an additive (b), i.e. a water-soluble polyanionic polymer, again results in a distinct reduction of the inhibitory effect of blood or plasma on the PCR amplification. At that, employing a water-soluble polyanionic polymer with a higher average molecular weight is advantageous, as may be seen from the comparison of 250,000 Da (LS 3) and 100,000 Da (LS 5).

### Example 3: Appearance of blood lysates in comparison

For blood lysis, 985 µl of LS 2 or LS 3, 5 µl of human blood and 10 µl of E. coli cell suspension (= 3 x 10⁶ cells of strain O157:H7) were mixed, incubated for 5 min at 95 °C under constant shaking at 1,400 rpm and cooled down to room temperature. As a reference, LS 2 was mixed only with E. coli cell suspension.

### Results:

The appearance of the lysates is shown in Fig. 5. The photography on the left hand shows the reference without blood, the photography in the middle shows the blood lysate not containing additive (b) and the photography on the right hand shows the blood lysate containing additive (b). The lysis solutions containing polyacrylic acid (additive (b)) show another advantage concerning the lysis of blood. Using these lysis solutions results in that after lysis blood constituents are still dissolved and red sediment is not formed. This is advantageous, if the lysate shall be used in PCR.

### Example 4: Influence of the used polymer on the depletion efficiency

Four different aqueous Ivsis compositions were tested:

| **Buffer 1 (B1)** | **Buffer 2 (B2)** | **Buffer 3 (B3)** | **Buffer 4 (B4)** |
|---|---|---|---|
| 0.1 % PVP (MW 10.000) | | 0.1 % PVP (MW 10.000) | 0.04 % PAA (MW 250k) |
| 0.45 % Tween-20 | 0.45 % Tween-20 | 0.45 % Tween-20 | 0.45 % Tween-20 |
| 0.45 % NP40 | 0.45 % NP40 | 0.45 % NP40 | 0.45 % NP40 |
| 40 mM Tris/Cl, pH 9 | 40 mM Tris/Cl, pH 9 | 40 mM borate, pH 9 | 40 mM Tris/Cl, pH 9 |
| 1 mM EDTA | 1 mM EDTA | 1 mM EDTA | 1 mM EDTA |

In separate reaction tubes comprising each 625 µg of Sera Mag Beads, 10 µm sections from fixed paraffin embedded rat liver tissue (PFPE - Paxgene-fixed, paraffin-embedded; fixation described in WO 2008/104564) were lysed in 250 µl of buffers 1 to 4. In addition, tissue samples were also lysed in presence of buffer 4 but without the addition of the carboxylated beads. All reactions were performed in duplicates. For lysis, the samples were incubated for 15 min at 95°C using a thermomixer, followed by about 5 minutes cooling to room temperature. Beads were separated using magnetic force for about 1 minute, and the supernatant (without the paraffin layer) which corresponds to the cleared lysis mixture was transferred to a new tube. The lysis mixture was analyzed by PCR using ß-actin as target. For PCR detection, the following reaction mixture was used: 12.5 µl QF Probe MM; 1 µl fwd primer (10 µM); 1 µl rev primer (10 µM); 1.25 µl probe (10 µM); 5 µl lysate; 4.25 µl water. PCR was performed using 95°C for 5 min (initial denaturation), followed by 40 cycles of denaturation at 95°C for 10 seconds, annealing at 55°C for 30 seconds and extension at 72°C for 30 seconds.

### Results:

The results are shown in Fig. 6. It was found that the lysis buffer 3 (B3) comprising borate as buffering agent yielded a better PCR performance than lysis buffer 1 (B1) comprising Tris, as can be seen by the lower Ct value. Comparing the results obtained for lysis buffer 4 (B4) (comprising polyacrylic acid (PAA) and thus a water-soluble polyanionic polymer comprising carboxylate-containing monomers) and lysis buffer 1 (B1) (comprising PVP), lysis performance of lysis buffer 4 (B4) was better. In addition, inhibitors were considerably more efficiently depleted when using lysis buffer 4 (B4) as compared to lysis buffer 1 (B1). This shows that PAA is more effective in depleting inhibitors than PVP. Better results were obtained with PAA even if no carboxylated beads were used for additional binding of inhibitors and/or removing precipitates. Thus, PAA alone was shown to be more effective than even the combination of PVP and carboxylated beads. However, to additionally clear the lysate from precipitates can be advantageous and thus, is preferred. Furthermore, the results obtained with buffer 2 (B2) show that also the polycarboxylated support alone has some PCR inhibitor depleting effect. However, the polymer(s) alone and also the combined use with the polymer(s) achieved significantly better results.

## Claims

1. A method for lysing a sample to provide a lysis mixture that is suitable for being directly used in a nucleic acid analysis method, wherein the sample is contacted with
(a) at least one non-ionic surfactant,
(b) at least one water-soluble polyanionic polymer, wherein said polymer comprises carboxylate-containing monomers, wherein said polymer is
(i) a polymer consisting of carboxylate-containing monomers that are selected from acrylic acid, methacrylic acid and/or maleic acid, or
(ii) a polymer consisting of carboxylate-containing monomers that are selected from acrylic acid, methacrylic acid and/or maleic acid and additional polar monomers without a carboxylate function that are selected from lactide and/or vinylpyrrolidone,
optionally wherein said polymer is a polymer selected from polyacrylic acid, polymaleic acid, polymethacrylic acid, poly(acrylic acid-co-maleic acid), poly(acrylic acid-co-lactide), poly(acrylic acid-co-vinylpyrrolidone), poly(maleic acid-co-lactide), poly(maleic acid-co-vinylpyrrolidone), poly(acrylic acid-co-maleic acid-co-lactide) and poly(acrylic acid-co-maleic acid-co-vinylpyrrolidone),
to provide a lysis mixture,
wherein the sample is contacted prior to, during or after lysis with at least one magnetic solid support which binds precipitates, thereby forming a complex with the precipitates.

2. The method according to claim 1, **wherein** the sample is contacted additionally with one or more of the following:
(c) at least one proteolytic enzyme;
(d) at least one chelating agent for divalent cations; and/or
(e) at least one buffer substance.

3. The method according to claim 1 or 2, **wherein** the at least one water-soluble polyanionic polymer has one or more of the following characteristics:
i) said polymer comprises acrylic acid and maleic acid, preferably consists of acrylic acid and maleic acid;
ii) said polymer comprises acrylic acid, preferably consists of acrylic acid;
iii) said polymer comprises maleic acid, preferably consists of maleic acid;
iv) said polymer is a block copolymer;
v) the at least one water-soluble polyanionic polymer is selected from polyacrylic acid, polymaleic acid and a co-polymer comprising polyacrylic acid and polymaleic acid; and/or
vi) the at least one water-soluble polyanionic polymer has an average molecular weight that is selected from a range of 2,000 Da to 300,000 Da, 10,000 to 450,000 Da, 25,000 to 400,000 Da, 35,000 to 350,000 Da, 50,000 Da to 300,000 Da, 100,000 Da to 300.000 Da and 200,000 Da to 300,000 Da.

4. The method according to one or more of claims 1 to 3, **wherein** the additives (a), (b), (c), (d) and/or (e) are provided in a lysis composition, preferably a lysis solution.

5. The method according to claim 4, having one or more, preferably at least three, more preferred at least four, more preferred at least five of the following characteristics:
a) the at least one non-ionic surfactant or a mixture of non-ionic surfactants is comprised in the lysis composition in a concentration selected from a range of 0.05% (v/v) to 5% (v/v), 0.1% (v/v) to 2% (v/v), 0.2% (v/v) to 1.5% (v/v) and 0.4% (v/v) to 1% (v/v);
b) the non-ionic surfactant or mixture of non-ionic surfactants is selected from the group consisting of polyoxyethylene fatty alcohol ethers, polyoxyethylene alkylphenyl ethers, polyoxyethylene-polyoxypropylene block copolymers, alkylglucosides and alkylphenol ethoxylates, preferably polyoxyethylene fatty alcohol ethers, polysorbates and/or polyoxyethylene alkyl phenyl ethers;
c) the at least one water-soluble polyanionic polymer or mixture of water-soluble polyanionic polymers is comprised in the lysis solution in a concentration that is selected from a range of 0.01% (w/v) to 1% (w/v), 0.02% (w/v) to 0.5% (w/v), 0.03% (w/v) to 0.3% (w/v), 0.04% (w/v) to 0.2% (w/v), 0.05% (w/v) to 0.15% (w/v) and 0.05% (w/v) to 0.1 % (w/v);
d) the proteolytic enzyme is a thermophilic proteinase;
e) the at least one chelating agent is selected from the group consisting of diethylenetriaminepentaacetic acid (DTPA), ethylenedinitrilotetraacetic acid (EDTA), ethylene glycol tetraacetic acid (EGTA) and N,N-bis(carboxymethyl)glycine (NTA);
f) the at least one chelating agent is comprised in the lysis composition in a concentration selected from a range of 0.5mM to 5mM, 0.75mM to 2mM and 1mM to 1.5mM;
g) the buffer substance is used in the lysis composition so that the pH of the composition is in a range selected from 7.5 and 11, 7.5 to 9 and 8,0 to 8,5; and/or
h) the buffer substance is comprised in the lysis composition in a concentration selected from 5mM to 100mM, 10mM to 80mM and 10mM to 50 mM.

6. The method according to claim 4 or 5, **wherein** the sample is contacted with a lysis composition comprising
- at least one non-ionic surfactant,
- at least one water-soluble polyanionic polymer comprising carboxylate-containing monomers which is selected from polyacrylic acid, polymaleic acid and a co-polymer comprising polyacrylic acid and polymaleic acid, wherein said polymer has an average molecular weight that lies in a range from 100,000 Da to 300.000 Da, preferably 200,000 Da to 300,000 Da,
- at least one buffer substance
wherein the pH of the lysis composition is in a range selected from 7.5 to 11, 7.5 to 9 and 8.0 to 8.5
to provide a lysis mixture.

7. The method according to one or more of claims 1 to 6, having one or more, preferably at least two or more preferred at least three of the following characteristics:
i) the sample is pretreated prior to contacting the sample with additives (a) and/or (b);
ii) the lysis mixture is incubated;
iii) the lysis mixture is incubated at a temperature selected from at least 40 °C, at least 50 °C, at least 90 °C and at least 95 °C;
iv) the lysis mixture comprises the lysed sample;
v) the lysis mixture is centrifuged; and/or
vi) the lysis mixture is cleared preferably by binding precipitates to a solid support.

8. The method according to one or more of claims 1 to 7, for lysing a fixed sample to provide a lysis mixture that is suitable for being directly used in a nucleic acid analysis method, wherein the fixed sample is contacted with
(a) at least one non-ionic surfactant,
(b) at least one water-soluble polyanionic polymer comprising carboxylate-containing monomers, *wherein said polymer is*
*(i) a polymer consisting of carboxylate-containing monomers that are selected from acrylic acid, methacrylic acid and*/*or maleic acid, or*
*(ii) a polymer consisting of carboxylate-containing monomers that are selected from acrylic acid, methacrylic acid and*/*or maleic acid and additional polar monomers without a carboxylate function that are selected from lactide and*/*or vinylpyrrolidone,*
preferably **wherein said polymer** is polyacrylic acid,
and is heated at ≥ 85 °C to provide a lysis mixture,
the method optionally comprising
a. contacting the fixed biological sample with
- an aqueous lysis composition having a pH value that lies in a range selected from 7.5 to 11, 7.5 to 9 and 8 to 8.5, comprising
(a) at least one non-ionic surfactant,
(b) at least one water-soluble polyanionic polymer comprising carboxylate-containing **monomers** in a concentration of 0.01 % (w/v) to 0.5% (w/v), preferably 0.03% (w/v) to 0.15% (w/v), *wherein said polymer is*
*(i) a polymer consisting of carboxylate-containing monomers that are selected from acrylic acid, methacrylic acid and*/*or maleic acid, or*
*(ii) a polymer consisting of carboxylate-containing monomers that are selected from acrylic acid, methacrylic acid and*/*or maleic acid and additional polar monomers without a carboxylate function that are selected from lactide and*/*or vinylpyrrolidone,*
(c) at least one buffering agent; and
(d) optionally at least one chelating agent;
and
- a solid support comprising anionic groups, preferably carboxyl groups, on the surface,
b. heating at a temperature of ≥ 90 °C, preferably ≥ 95 °C to provide a lysis mixture and optionally cooling the lysis mixture;
c. separating the solid support from the remaining lysis mixture.

9. A nucleic acid amplification method, comprising
a) lysing a sample comprising nucleic acids to provide a lysis mixture that is suitable for being directly used in a nucleic acid analysis method, wherein the sample is contacted with
(aa) at least one non-ionic surfactant,
(ab) at least one water-soluble polyanionic polymer, wherein said polymer comprises carboxylate-containing monomers, wherein said polymer is
(i) a polymer consisting of carboxylate-containing monomers that are selected from acrylic acid, methacrylic acid and/or maleic acid, or
(ii) a polymer consisting of carboxylate-containing monomers that are selected from acrylic acid, methacrylic acid and/or maleic acid and additional polar monomers without a carboxylate function that are selected from lactide and/or vinylpyrrolidone,
optionally wherein said polymer is a polymer selected from polyacrylic acid, polymaleic acid, polymethacrylic acid, poly(acrylic acid-co-maleic acid), poly(acrylic acid-co-lactide), poly(acrylic acid-co-vinylpyrrolidone), poly(maleic acid-co-lactide), poly(maleic acid-co-vinylpyrrolidone), poly(acrylic acid-co-maleic acid-co-lactide) and poly(acrylic acid-co-maleic acid-co-vinylpyrrolidone),
to provide a lysis mixture, preferably wherein the sample is lysed according to the method as defined in one or more of claims 1 to 8;
b) using at least a portion of the lysis mixture in an amplification reaction.

10. The method according to claim 9, having one or more of the following characteristics:
i) the lysis mixture is cleared between steps a) and b) and a portion of the cleared lysis mixture is used in an amplification reaction;
ii) no nucleic acid isolation method is performed between steps a) and b);
iii) the lysate is processed between step a) and b) by contacting the obtained lysate with at least one enzyme;
iv) the obtained lysate is used to reconstitute a dry composition, preferably a freeze-dried composition, which comprises reagents for performing the amplification; and/or
v) the amplification reaction is selected from the group consisting of polymerase chain reaction (PCR), isothermal amplification, reverse transcription polymerase chain reaction (RT-PCR), reverse transcription amplification, quantitative real time polymerase chain reaction (qPCR), DNA or RNA sequencing, reverse transcription, LAMP (loop mediated isothermal amplification), RPA (recombinase polymerase amplification), tHDA (helix dependent amplification), NEAR (nicking enzyme amplification reaction), TMA (transcription mediated amplification) and NASBA (nucleic acid sequence based amplification), allele specific polymerase chain reaction, polymerase cycling assembly (PCA), asymmetric polymerase chain reaction, linear after the exponential polymerase chain reaction (LATE-PCR), helicase-dependent amplification (HDA), hot-start polymerase chain reaction, intersequence-specific polymerase chain reaction (ISSR), inverse polymerase chain reaction, ligation mediated polymerase chain reaction, methylation specific polymerase chain reaction (MSP), multiplex polymerase chain reaction, nested polymerase chain reaction and solid phase polymerase chain reaction.

11. A lysis solution suitable to provide a lysis mixture that can be directly used in a nucleic acid amplification, comprising
(a) at least one non-ionic surfactant,
(b) at least one water-soluble polyanionic polymer comprising carboxylate-containing monomers, wherein the at least one water-soluble polyanionic polymer or mixture of water-soluble polyanionic polymers is comprised in the lysis solution in a concentration of 0.01 % (w/v) to 1% (w/v), and
wherein said polymer is
(i) a polymer consisting of carboxylate-containing monomers that are selected from acrylic acid, methacrylic acid and/or maleic acid, or
(ii) a polymer consisting of carboxylate-containing monomers that are selected from acrylic acid, methacrylic acid and/or maleic acid and additional polar monomers without a carboxylate function that are selected from lactide and/or vinylpyrrolidone,
optionally wherein said polymer is a polymer selected from polyacrylic acid, polymaleic acid, polymethacrylic acid, poly(acrylic acid-co-maleic acid), poly(acrylic acid-co-lactide), poly(acrylic acid-co-vinylpyrrolidone), poly(maleic acid-co-lactide), poly(maleic acid-co-vinylpyrrolidone), poly(acrylic acid-co-maleic acid-co-lactide) and poly(acrylic acid-co-maleic acid-co-vinylpyrrolidone).

12. The lysis solution according to claim 11, additionally comprising
(c) at least one proteolytic enzyme;
(d) at least one chelating agent for divalent cations; and/or
(e) at least one buffer substance.

13. The lysis solution according to claim 11 or 12, wherein the at least one water-soluble polyanionic polymer comprising carboxylate-containing monomers is selected from polyacrylic acid, polymaleic acid and a co-polymer comprising polyacrylic acid and polymaleic acid and wherein said polymer has an average molecular weight that lies in a range from 100,000 Da to 300.000 Da, preferably 200,000 Da to 300,000 Da, and wherein the pH of the lysis solution is in a range selected from 7.5 to 11, 7.5 to 9 and 8.0 to 8.5

14. The lysis solution according to one or more of claims 11 to 13, having one or more, preferably at least two, at least three, at least four and more preferred at least five of the following characteristics:
a) the at least one water-soluble polyanionic polymer has one or more of the characteristics as defined in claim 3;
b) the at least one non-ionic surfactant or mixture of non-ionic surfactants is comprised in the lysis solution in a concentration selected from a range of 0.05% (v/v) to 5% (v/v), 0.1 % (v/v) to 2% (v/v), 0.2% (v/v) to 1.5% (v/v) and 0.4% (v/v) to 1 % (v/v);
c) the non-ionic surfactant or mixture of non-ionic surfactants is selected from the group consisting of polyoxyethylene fatty alcohol ethers, polyoxyethylene alkylphenyl ethers, polyoxyethylene-polyoxypropylene block copolymers, alkylglucosides and alkylphenol ethoxylates, preferably polyoxyethylene fatty alcohol ethers, polysorbates and/or polyoxyethylene alkyl phenyl ethers;
d) the at least one water-soluble polyanionic polymer or mixture of water-soluble polyanionic polymers is comprised in the lysis solution in a concentration that is selected from a range of 0.02% (w/v) to 0.5% (w/v), 0.03% (w/v) to 0.3% (w/v), 0.04% (w/v) to 0.2% (w/v), 0.05% (w/v) to 0.15% (w/v) and 0.05% (w/v) to 0.1 % (w/v) and wherein, preferably, the at least one water-soluble polyanionic polymer is selected from polyacrylic acid, polymaleic acid and a co-polymer comprising polyacrylic acid and polymaleic acid and more preferred is or comprises polyacrylic acid;
e) the at least one water-soluble polyanionic polymer which is or comprises polyacrylic acid has an average molecular weight that lies in a range from 100,000 Da to 300.000 Da, preferably 200,000 Da to 300,000 Da;
f) the proteolytic enzyme is a thermophilic proteinase;
g) the at least one chelating agent is selected from the group consisting of diethylenetriaminepentaacetic acid (DTPA), ethylenedinitrilotetraacetic acid (EDTA), ethylene glycol tetraacetic acid (EGTA) and N,N-bis(carboxymethyl)glycine (NTA);
h) the at least one chelating agent is comprised in the lysis solution in a concentration selected from a range of 0.5mM to 5mM, 0.75mM to 2mM and 1 mM to 1.5mM;
i) the buffer substance is used in the lysis solution so that the pH of the solution is selected from a range of 7.5 and 11, 7.5 to 9 and 8 to 8.5;
j) the buffer substance is comprised in the lysis solution in a concentration selected from a range of 5mM to 100mM, 10mM to 80mM and 10 to 50 mM; and/or
k) the lysis solution is a lysis solution comprising
- as additive (a) a mixture of two non-ionic detergents, wherein each non-ionic detergent is comprised in a concentration of 0.2% (v/v) to 0.6% (v/v), preferably 0.4% (v/v) to 0.5% (v/v);
- as additive (b), a water-soluble polyanionic polymer, which preferably is or comprises polyacrylic acid, in a concentration of 0.01% (w/v) to 0.5% (w/v), preferably 0.03% (w/v) to 0.15% (w/v);
- optionally a proteolytic enzyme such as proteinase K as additive (c);
- as additive (d) EDTA in a concentration below 1.5mM; and
- as additive (e) a buffer substance, preferably TRIS, in a concentration selected from 7.5mM to 50mM.

15. The lysis solution as defined in any one of claims 11 to 14, being mixed with a nucleic acid containing sample.

## Patentansprüche

1. Verfahren zum Lysieren einer Probe, um eine Lysemischung bereitzustellen, die geeignet ist, um direkt in einem Nukleinsäureanalyseverfahren verwendet zu werden, wobei die Probe in Kontakt gebracht wird mit:
(a) zumindest einem nichtionischen Tensid,
(b) zumindest einem wasserlöslichen polyanionischen Polymer, wobei das Polymer Carboxylat enthaltende Monomere aufweist, wobei das Polymer Folgendes ist:
(i) ein Polymer, das aus Carboxylat enthaltenden Monomeren besteht, die aus Acrylsäure, Methacrylsäure und/oder Maleinsäure ausgewählt sind, oder
(ii) ein Polymer, das aus Carboxylat enthaltenden Monomeren, die aus Acrylsäure, Methacrylsäure und/oder Maleinsäure ausgewählt sind, und aus zusätzlichen polaren Monomeren ohne eine Carboxylatfunktion, die aus Lactid und/oder Vinylpyrrolidon ausgewählt sind, besteht,
wobei das Polymer optional ein aus Polyacrylsäure, Polymaleinsäure, Polymethacrylsäure, Poly(acrylsäure-co-maleinsäure), Poly(acrylsäure-co-lactid), Poly(acrylsäure-co-vinylpyrrolidon), Poly(maleinsäure-co-lactid), Poly(maleinsäure-co-vinylpyrrolidon), Poly(acrylsäure-co-maleinsäure-co-lactid) und Poly(acrylsäure-co-maleinsäure-co-vinylpyrrolidon) ausgewähltes Polymer ist,
um eine Lysemischung bereitzustellen,
wobei die Probe vor, während oder nach der Lyse mit zumindest einem magnetischen festen Träger in Kontakt gebracht wird, der Präzipitate bindet, wodurch ein Komplex mit den Präzipitaten gebildet wird.

2. Verfahren nach Anspruch 1, **wobei** die Probe zusätzlich mit einem oder mehreren der Folgenden in Kontakt gebracht wird:
(c) zumindest einem proteolytischen Enzym;
(d) zumindest einem Chelatbildner für divalente Kationen; und/oder
(e) zumindest einer Puffersubstanz.

3. Verfahren nach Anspruch 1 oder 2, wobei das zumindest eine wasserlösliche polyanionische Polymer eines oder mehrere der folgenden Merkmale aufweist:
i) das Polymer umfasst Acrylsäure und Maleinsäure, wobei es vorzugsweise aus Acrylsäure und Maleinsäure besteht;
ii) das Polymer umfasst Acrylsäure, wobei es vorzugsweise aus Acrylsäure besteht;
iii) das Polymer umfasst Maleinsäure, wobei es vorzugsweise aus Maleinsäure besteht;
iv) das Polymer ist ein Blockcopolymer;
v) das zumindest eine wasserlösliche polyanionische Polymer ist aus Polyacrylsäure, Polymaleinsäure und einem Copolymer ausgewählt, das Polyacrylsäure und Polymaleinsäure umfasst; und/oder
vi) das zumindest eine wasserlösliche polyanionische Polymer weist ein mittleres Molekulargewicht auf, das aus einem Bereich von 2.000 Da bis 300.000 Da, 10.000 bis 450.000 Da, 25.000 bis 400.000 Da, 35.000 bis 350.000 Da, 50.000 Da bis 300.000 Da, 100.000 Da bis 300.000 Da und 200.000 Da bis 300.000 Da ausgewählt ist.

4. Verfahren nach einem oder mehreren der Ansprüche 1 bis 3, wobei die Zusatzstoffe (a), (b), (c), (d) und/oder (e) in einer Lysezusammensetzung, vorzugsweise in einer Lyselösung, bereitgestellt werden.

5. Verfahren nach Anspruch 4, das eines oder mehrere, vorzugsweise zumindest drei, bevorzugter zumindest vier, bevorzugter zumindest fünf, der folgenden Merkmale aufweist:
a) das zumindest eine nichtionische Tensid oder eine Mischung aus nichtionischen Tensiden ist in einer aus einem Bereich von 0,05 % (v/v) bis 5 % (v/v), 0,1 % (v/v) bis 2 % (v/v), 0,2 % (v/v) bis 1,5 % (v/v) und 0,4 % (v/v) bis 1 % (v/v) ausgewählten Konzentration in der Lysezusammensetzung enthalten;
b) das nichtionische Tensid oder die Mischung aus nichtionischen Tensiden ist aus der Gruppe bestehend aus Polyoxyethylenfettalkoholethern, Polyoxyethylenalkylphenylethern, Polyoxyethylen-Polyoxypropylen-Blockcopolymeren, Alkylglucosiden und Alkylphenolethoxylaten ausgewählt, vorzugsweise aus Polyoxyethylenfettalkoholethern, Polysorbaten und/oder Polyoxyethylenalkylphenylethern;
c) das zumindest eine wasserlösliche polyanionische Polymer oder die Mischung aus wasserlöslichen polyanionischen Polymeren ist in einer aus einem Bereich von 0,01 % (w/v) bis 1 % (w/v), 0,02 % (w/v) bis 0,5 % (w/v), 0,03 % (w/v) bis 0,3 % (w/v), 0,04 % (w/v) bis 0,2 % (v/v), 0,05 % (w/v) bis 0,15 % (w/v) und 0,05 % (w/v) bis 0,1 % (w/v) ausgewählten Konzentration in der Lyselösung enthalten;
d) das proteolytische Enzym ist eine thermophile Proteinase;
e) der zumindest eine Chelatbildner ist aus der Gruppe bestehend aus Diethylentriaminpentaessigsäure (DTPA), Ethylendinitrilotetraessigsäure (EDTA), Ethylenglykoltetraessigsäure (EGTA) und N,N-Bis(carboxymethyl)glycin (NTA) ausgewählt;
f) der zumindest eine Chelatbildner ist in einer aus einem Bereich von 0,5 mM bis 5 mM, 0,75 mM bis 2 mM und 1 mM bis 1,5 mM ausgewählten Konzentration in der Lysezusammensetzung enthalten;
g) die Puffersubstanz wird in der Lysezusammensetzung derart verwendet, dass der pH-Wert der Zusammensetzung in einem Bereich liegt, der ausgewählt ist aus von 7,5 und 11, von 7,5 bis 9 und von 8,0 bis 8,5; und/oder
h) die Puffersubstanz ist in einer aus von 5 mM bis 100 mM, von 10 mM bis 80 mM und von 10 mM bis 50 mM ausgewählten Konzentration in der Lysezusammensetzung vorhanden.

6. Verfahren nach Anspruch 4 oder 5, wobei die Probe mit einer Lysezusammensetzung in Kontakt gebracht wird, die Folgendes umfasst:
- zumindest ein nichtionisches Tensid,
- zumindest ein wasserlösliches polyanionisches Polymer, das Carboxylat enthaltende Monomere umfasst, das aus Polyacrylsäure, Polymaleinsäure und einem Copolymer ausgewählt ist, das Polyacrylsäure und Polymaleinsäure umfasst, wobei das Polymer ein mittleres Molekulargewicht aufweist, das in einem Bereich von 100.000 Da bis 300.000 Da, vorzugsweise von 200.000 Da bis 300.000 Da, liegt,
- zumindest eine Puffersubstanz
wobei der pH-Wert der Lysezusammensetzung in einem Bereich liegt, der ausgewählt ist aus von 7,5 bis 11, 7,5 bis 9 und 8,0 bis 8,5,
um eine Lysemischung bereitzustellen.

7. Verfahren nach einem oder mehreren der Ansprüche 1 bis 6, das eines oder mehr, vorzugsweise zumindest zwei, stärker bevorzugt zumindest drei, der folgenden Merkmale aufweist:
i) die Probe wird vorbehandelt, bevor die Probe mit den Zusatzstoffen (a) und/oder (b) in Kontakt gebracht wird;
ii) die Lysemischung wird inkubiert;
iii) die Lysemischung wird bei einer Temperatur inkubiert, die ausgewählt ist aus mindestens 40 °C, mindestens 50 °C, mindestens 90 °C und mindestens 95 °C;
iv) die Lysemischung umfasst die lysierte Probe;
v) die Lysemischung wird zentrifugiert; und/oder
vi) die Lysemischung wird geklärt, vorzugsweise durch Binden von Präzipitaten an einen festen Träger.

8. Verfahren nach einem oder mehreren der Ansprüche 1 bis 7 zum Lysieren einer fixierten Probe, um eine Lysemischung bereitzustellen, die geeignet ist, um direkt in einem Nukleinsäureanalyseverfahren verwendet zu werden, wobei die fixierte Probe in Kontakt gebracht wird mit:
(a) zumindest einem nichtionischen Tensid,
(b) zumindest einem wasserlöslichen polyanionischen Polymer, das Carboxylat enthaltende Monomere umfasst, wobei das Polymer Folgendes ist (i) ein Polymer, das aus Carboxylat enthaltenden Monomeren besteht, die aus Acrylsäure, Methacrylsäure und/oder Maleinsäure ausgewählt sind, oder (ii) ein Polymer, das aus Carboxylat enthaltenden Monomeren, die aus Acrylsäure, Methacrylsäure und/oder Maleinsäure ausgewählt sind, und aus zusätzlichen polaren Monomeren ohne eine Carboxylatfunktion, die aus Lactid und/oder Vinylpyrrolidon ausgewählt sind, besteht, vorzugsweise wobei das Polymer Polyacrylsäure ist,
und bei ≥ 85 °C erhitzt wird, um eine Lysemischung bereitzustellen, wobei das Verfahren optional Folgendes umfasst:
a. Inkontaktbringen der fixierten biologischen Probe mit:
- einer wässrigen Lysezusammensetzung, die einen pH-Wert aufweist, der in einem aus von 7,5 bis 11, von 7,5 bis 9 und von 8 bis 8,5 ausgewählten Bereich liegt, die Folgendes umfasst:
(a) zumindest ein nichtionisches Tensid,
(b) zumindest ein wasserlösliches polyanionisches Polymer, das Carboxylat enthaltende Monomere in einer Konzentration von 0,01 % (w/v) bis 0,5 % (w/v), vorzugsweise von 0,03 % (w/v) bis 0,15 % (w/v) umfasst, wobei das Polymer Folgendes ist (i) ein Polymer, das aus Carboxylat enthaltenden Monomeren besteht, die aus Acrylsäure, Methacrylsäure und/oder Maleinsäure ausgewählt sind, oder (ii) ein Polymer, das aus Carboxylat enthaltenden Monomeren, die aus Acrylsäure, Methacrylsäure und/oder Maleinsäure ausgewählt sind, und aus zusätzlichen polaren Monomeren ohne eine Carboxylatfunktion, die aus Lactid und/oder Vinylpyrrolidon ausgewählt sind, besteht,
(c) zumindest ein Puffersubstanz, und
(d) optional zumindest einen Chelatbildner;
und
- einem festen Träger, der anionische Gruppen, vorzugsweise Carboxylgruppen, auf der Oberfläche umfasst,
b. Erhitzen bei einer Temperatur von ≥ 90 °C, vorzugsweise von ≥ 95 °C, um eine Lysemischung bereitzustellen, und optional Abkühlen der Lysemischung;
c. Abtrennen des festen Trägers von der übrigen Lysemischung.

9. Nukleinsäureamplifikationsverfahren, umfassend:
a) Lysieren einer Probe, die Nukleinsäuren umfasst, um eine Lysemischung bereitzustellen, die geeignet ist, um direkt in einem Nukleinsäureanalyseverfahren verwendet zu werden, wobei die Probe in Kontakt gebracht wird mit:
(aa) zumindest einem nichtionischen Tensid,
(ab) zumindest einem wasserlöslichen polyanionischen Polymer, wobei das Polymer Carboxylat enthaltende Monomere umfasst, wobei das Polymer Folgendes ist:
(i) ein Polymer, das aus Carboxylat enthaltenden Monomeren besteht, die aus Acrylsäure, Methacrylsäure und/oder Maleinsäure ausgewählt sind, oder
(ii) ein Polymer, das aus Carboxylat enthaltenden Monomeren, die aus Acrylsäure, Methacrylsäure und/oder Maleinsäure ausgewählt sind, und aus zusätzlichen polaren Monomeren ohne eine Carboxylatfunktion, die aus Lactid und/oder Vinylpyrrolidon ausgewählt sind, besteht,
wobei das Polymer optional ein aus Polyacrylsäure, Polymaleinsäure, Polymethacrylsäure, Poly(acrylsäure-co-maleinsäure), Poly(acrylsäure-co-lactid), Poly(acrylsäure-co-vinylpyrrolidon), Poly(maleinsäure-co-lactid), Poly(maleinsäure-co-vinylpyrrolidon), Poly(acrylsäure-co-maleinsäure-co-lactid) und Poly(acrylsäure-co-maleinsäure-co-vinylpyrrolidon) ausgewähltes Polymer ist,
um eine Lysemischung bereitzustellen, wobei die Probe vorzugsweise nach dem in einem oder mehreren der Ansprüche 1 bis 8 definierten Verfahren lysiert wird;
b) Verwenden von zumindest einem Teil der Lysemischung in einer Amplifikationsreaktion.

10. Verfahren nach Anspruch 9, das eines oder mehrere der folgenden Merkmale aufweist:
i) die Lysemischung wird zwischen den Schritten a) und b) geklärt und ein Teil der geklärten Lysemischung wird in einer Amplifikationsreaktion verwendet;
ii) es wird kein Nukleinsäureisolierungsverfahren zwischen den Schritten a) und b) durchgeführt;
iii) das Lysat wird zwischen den Schritten a) und b) verarbeitet, indem das erhaltene Lysat mit zumindest einem Enzym in Kontakt gebracht wird;
iv) das erhaltene Lysat wird verwendet, um eine Trockenzusammensetzung, vorzugsweise eine gefriergetrocknete Zusammensetzung, die Reagenzien umfasst, um die Amplifikation durchzuführen, zu rekonstituieren; und/oder
v) die Amplifikationsreaktion ist ausgewählt aus der Gruppe bestehend aus: Polymerase-Kettenreaktion (PCR), isothermer Amplifikation, Reverse-Transkription-Polymerase-Kettenreaktion (RT-PCR), reverser Transkription-Amplifikation, quantitativer Echtzeit-Polymerase-Kettenreaktion (qPCR), DNA- oder RNA-Sequenzierung, reverser Transkription, LAMP (Schleifen-vermittelte isothermale Amplifikation), RPA (Rekombinase-Polymerase-Amplifikation), tHDA (Helix-abhängige Amplifikation), NEAR (Einzelstrangbruchsenzym-Amplifikationsreaktion), TMA (Transkriptions-vermittelte Amplifikation) und NASBA (Nukleinsäuresequenz-basierte Amplifikation), allelspezifischer Polymerase-Kettenreaktion, Polymerase Cycling Assembly (PCA), asymmetrischer Polymerase-Kettenreaktion, Linear-After-The-Exponential-Polymerase-Chain-Reaction (LATE-PCR), Helikase-abhängiger Amplifikation (HDA), Hot-Start-Polymerase-Kettenreaktion, intersequenzspezifischer Polymerase-Kettenreaktion (ISSR), inverser Polymerase-Kettenreaktion, ligationsvermittelter Polymerase-Kettenreaktion, Methylierungs-spezifischer Polymerase-Kettenreaktion (MSP), Multiplex-Polymerase-Kettenreaktion, verschachtelter Polymerase-Kettenreaktion und Festphasen-Polymerase-Kettenreaktion.

11. Lyselösung, die geeignet ist, um eine Lysemischung bereitzustellen, die direkt in einer Nukleinsäureamplifikation verwendet werden kann, umfassend:
(a) zumindest ein nichtionisches Tensid,
(b) zumindest ein wasserlösliches polyanionisches Polymer, das Carboxylat enthaltende Monomere umfasst, wobei das zumindest eine wasserlösliche polyanionische Polymer oder die Mischung aus wasserlöslichen polyanionischen Polymeren in einer Konzentration von 0,01 % (w/v) bis 1 % (w/v) in der Lyselösung enthalten ist und
wobei das Polymer Folgendes ist:
(i) ein Polymer, das aus Carboxylat enthaltenden Monomeren besteht, die aus Acrylsäure, Methacrylsäure und/oder Maleinsäure ausgewählt sind, oder
(ii) ein Polymer, das aus Carboxylat enthaltenden Monomeren, die aus Acrylsäure, Methacrylsäure und/oder Maleinsäure ausgewählt sind, und aus zusätzlichen polaren Monomeren ohne eine Carboxylatfunktion, die aus Lactid und/oder Vinylpyrrolidon ausgewählt sind, besteht,
wobei das Polymer optional ein aus Polyacrylsäure, Polymaleinsäure, Polymethacrylsäure, Poly(acrylsäure-co-maleinsäure), Poly(acrylsäure-co-lactid), Poly(acrylsäure-co-vinylpyrrolidon), Poly(maleinsäure-co-lactid), Poly(maleinsäure-co-vinylpyrrolidon), Poly(acrylsäure-co-maleinsäure-co-lactid) und Poly(acrylsäure-co-maleinsäure-co-vinylpyrrolidon) ausgewähltes Polymer ist.

12. Lyselösung nach Anspruch 11, ferner umfassend:
(c) zumindest ein proteolytisches Enzym;
(d) zumindest einen Chelatbildner für divalente Kationen; und/oder
(e) zumindest eine Puffersubstanz.

13. Lyselösung nach einem der Ansprüche 11 oder 12, wobei das zumindest eine wasserlösliche polyanionische Polymer, das Carboxylat enthaltende Monomere umfasst, aus Polyacrylsäure, Polymaleinsäure und einem Copolymer ausgewählt ist, das Polyacrylsäure und Polymaleinsäure umfasst, und wobei das Polymer ein mittleres Molekulargewicht aufweist, das in einem Bereich von 100.000 Da bis 300.000 Da, vorzugsweise von 200.000 Da bis 300.000 Da, liegt, und wobei der pH-Wert der Lyselösung in einem aus von 7,5 bis 11, von 7,5 bis 9 und von 8,0 bis 8,5 ausgewählten Bereich ist.

14. Lyselösung nach einem oder mehreren der Ansprüche 11 bis 13, die eines oder mehrere, vorzugsweise zumindest zwei, zumindest drei, zumindest vier und bevorzugter zumindest fünf, der folgenden Merkmale aufweist:
a) das zumindest eine wasserlösliche polyanionische Polymer weist eines oder mehrere der in Anspruch 3 definierten Merkmale auf;
b) das zumindest eine nichtionische Tensid oder die Mischung aus nichtionischen Tensiden ist in einer aus einem Bereich von 0,05 % (v/v) bis 5 % (v/v), 0,1 % (v/v) bis 2 % (v/v), 0,2 % (v/v) bis 1,5 % (v/v) und 0,4 % (v/v) bis 1 % (v/v) ausgewählten Konzentration in der Lyselösung enthalten;
c) das nichtionische Tensid oder die Mischung aus nichtionischen Tensiden ist aus der Gruppe bestehend aus Polyoxyethylenfettalkoholethern, Polyoxyethylenalkylphenylethern, Polyoxyethylen-Polyoxypropylen-Blockcopolymeren, Alkylglucosiden und Alkylphenolethoxylaten ausgewählt, vorzugsweise aus Polyoxyethylenfettalkoholethern, Polysorbaten und/oder Polyoxyethylenalkylphenylethern;
d) das zumindest eine wasserlösliche polyanionische Polymer oder die Mischung aus wasserlöslichen polyanionischen Polymeren ist in einer aus einem Bereich von 0,02 % (w/v) bis 0,5 % (w/v), 0,03 % (w/v) bis 0,3 % (w/v), 0,04 % (w/v) bis 0,2 % (w/v), 0,05 % (w/v) bis 0,15 % (v/v) und 0,05 % (w/v) bis 0,1 % (w/v) ausgewählten Konzentration in der Lyselösung enthalten, und wobei das zumindest eine wasserlösliche polyanionische Polymer vorzugsweise aus Polyacrylsäure, Polymaleinsäure und einem Copolymer ausgewählt ist, das Polyacrylsäure und Polymaleinsäure umfasst, und noch bevorzugter Polyacrylsäure ist oder umfasst;
e) das zumindest eine wasserlösliche polyanionische Polymer, das Polyacrylsäure ist oder umfasst, weist ein mittleres Molekulargewicht auf, das im Bereich von 100.000 Da bis 300.000 Da, vorzugsweise von 200.000 Da bis 300.000 Da, liegt;
f) das proteolytische Enzym ist eine thermophile Proteinase;
g) der zumindest eine Chelatbildner ist aus der Gruppe bestehend aus Diethylentriaminpentaessigsäure (DTPA), Ethylendinitrilotetraessigsäure (EDTA), Ethylenglykoltetraessigsäure (EGTA) und N,N-Bis(carboxymethyl)glycin (NTA) ausgewählt;
h) der zumindest eine Chelatbildner ist in einer aus einem Bereich von 0,5 mM bis 5 mM, 0,75 mM bis 2 mM und 1 mM bis 1,5 mM ausgewählten Konzentration in der Lyselösung enthalten;
i) die Puffersubstanz wird in der Lyselösung derart verwendet, dass der pH-Wert der Lösung aus einem Bereich von 7,5 und 11, von 7,5 bis 9 und von 8 bis 8,5 ausgewählt ist;
j) die Puffersubstanz ist in einer aus einem Bereich von 5 mM bis 100 mM, von 10 mM bis 80 mM und von 10 mM bis 50 mM ausgewählten Konzentration in der Lyselösung vorhanden; und/oder
k) die Lyselösung ist eine Lyselösung, die Folgendes umfasst:
- eine Mischung aus zwei nichtionischen Detergenzien als Zusatzstoff (a), wobei jedes nichtionische Detergens in einer Konzentration von 0,2 % (v/v) bis 0,6 % (v/v), vorzugsweise von 0,4 % (v/v) bis 0,5 % (v/v), enthalten ist;
- ein wasserlösliches polyanionisches Polymer als Zusatzstoff (b), das vorzugsweise Polyacrylsäure ist oder umfasst, in einer Konzentration von 0,01 % (w/v) bis 0,5 % (w/v), vorzugsweise von 0,03 % (w/v) bis 0,15 % (w/v);
- optional ein proteolytisches Enzym, wie etwa Proteinase K, als Zusatzstoff (c);
- EDTA in einer Konzentration unter 1,5 mM als Zusatzstoff (d); und
- eine Puffersubstanz, vorzugsweise TRIS, in einer aus 7,5 mM bis 50 mM ausgewählten Konzentration als Zusatzstoff (e).

15. Lyselösung wie in einem der Ansprüche 11 bis 14 definiert, die mit einer eine Nukleinsäure enthaltende Probe gemischt ist.

## Revendications

1. Procédé pour la lyse d'un échantillon pour fournir un mélange de lyse qui est approprié pour être directement utilisé dans un procédé d'analyse d'acides nucléiques, dans lequel l'échantillon est mis en contact avec
(a) au moins un tensioactif non ionique,
(b) au moins un polymère polyanionique hydrosoluble, ledit polymère comprenant des monomères contenant une fonction carboxylate, ledit polymère étant
(i) un polymère constitué de monomères contenant une fonction carboxylate qui sont choisis parmi l'acide acrylique, l'acide méthacrylique et/ou l'acide maléique ou
(ii) un polymère constitué de monomères contenant une fonction carboxylate qui sont choisis parmi l'acide acrylique, l'acide méthacrylique et/ou l'acide maléique et de monomères polaires supplémentaires sans fonction carboxylate qui sont choisis parmi le lactide et/ou la vinylpyrrolidone,
éventuellement ledit polymère étant un polymère choisi parmi les polymères poly(acide acrylique), poly(acide maléique), poly(acide méthacrylique), poly(acide acrylique-co-acide maléique), poly(acide acrylique-co-lactide), poly(acide acrylique-co-vinylpyrrolidone), poly(acide maléique-co-lactide), poly(acide maléique-co-vinylpyrrolidone), poly(acide acrylique-co-acide maléique-co-lactide) et poly(acide acrylique-co-acide maléique-co-vinylpyrrolidone),
pour fournir un mélange de lyse,
dans lequel l'échantillon est mis en contact avant, pendant ou après la lyse avec au moins un support solide magnétique qui se lie à des précipités, ce qui forme de cette manière un complexe avec les précipités.

2. Procédé selon la revendication 1, **dans lequel** l'échantillon est mis en contact de plus avec un ou plusieurs des composants suivants :
(c) au moins une enzyme protéolytique ;
(d) au moins un agent chélatant pour des cations divalents ; et/ou
(e) au moins une substance tampon.

3. Procédé selon la revendication 1 ou 2, **dans lequel** l'au moins un polymère polyanionique hydrosoluble a une ou plusieurs des caractéristiques suivantes :
i) ledit polymère comprend de l'acide acrylique et de l'acide maléique, de préférence est constitué d'acide acrylique et d'acide maléique ;
ii) ledit polymère comprend de l'acide acrylique, de préférence est constitué d'acide acrylique ;
iii) ledit polymère comprend de l'acide maléique, de préférence est constitué d'acide maléique ;
iv) ledit polymère est un copolymère séquencé ;
v) l'au moins un polymère polyanionique hydrosoluble est choisi parmi le poly(acide acrylique), le poly(acide maléique) et un copolymère comprenant du poly(acide acrylique) et du poly(acide maléique) ; et/ou
vi) l'au moins un polymère polyanionique hydrosoluble a une masse moléculaire moyenne qui est choisie dans une plage de 2 000 Da à 300 000 Da, de 10 000 à 450 000 Da, de 25 000 à 400 000 Da, de 35 000 à 350 000 Da, de 50 000 Da à 300 000 Da, de 100 000 Da à 300 000 Da et de 200 000 Da à 300 000 Da.

4. Procédé selon une ou plusieurs des revendications 1 à 3, **dans lequel** les additifs (a), (b), (c), (d) et/ou (e) sont fournis dans une composition de lyse, de préférence une solution de lyse.

5. Procédé selon la revendication 4, ayant une ou plusieurs, de préférence au moins trois, de préférence encore au moins quatre, de préférence encore au moins cinq des caractéristiques suivantes :
a) l'au moins tensioactif non ionique ou un mélange de tensioactifs non ioniques est compris dans la composition de lyse en une concentration choisie dans une plage de 0,05 % (v/v) à 5 % (v/v), de 0,1 % (v/v) à 2 % (v/v), de 0,2 % (v/v) à 1,5 % (v/v) et de 0,4 % (v/v) à 1 % (v/v) ;
b) le tensioactif non ionique ou le mélange de tensioactifs non ioniques est choisi dans le groupe constitué par les éthers d'alcools gras et de polyoxyéthylène, les éthers d'alkylphényle et de polyoxyéthylène, les copolymères séquencés de polyoxyéthylène-polyoxypropylène, les alkylglucosides et les produits d'éthoxylation d'alkylphénols, de préférence les éthers d'alcools gras et de polyoxyéthylène, les polysorbates et/ou les éthers d'alkylphényle et de polyoxyéthylène ;
c) l'au moins un polymère polyanionique hydrosoluble ou le mélange de polymères polyanioniques hydrosolubles est compris dans la solution de lyse en une concentration qui est choisie dans une plage de 0,01 % (p/v) à 1 % (p/v), de 0,02 % (p/v) à 0,5 % (p/v), de 0,03 % (p/v) à 0,3 % (p/v), de 0,04 % (p/v) à 0,2 % (p/v), de 0,05 % (p/v) à 0,15 % (p/v) et de 0,05 % (p/v) à 0,1 % (p/v) ;
d) l'enzyme protéolytique est une protéinase thermophile ;
e) l'au moins un agent chélatant est choisi dans le groupe constitué par l'acide diéthylènetriaminepentaacétique (DTPA), l'acide éthylènedinitrilotétraacétique (EDTA), l'acide éthylèneglycoltétraacétique (EGTA) et la *N,N*-bis(carboxyméthyl)glycine (NTA) ;
f) l'au moins un agent chélatant est compris dans la composition de lyse en une concentration choisie dans une plage de 0,5 mM à 5 mM, de 0,75 mM à 2 mM et de 1 mM à 1,5 mM ;
g) la substance tampon est utilisée dans la composition de lyse de façon à ce que le pH de la composition soit dans une plage choisie parmi 7,5 à 11, 7,5 à 9 et 8,0 à 8,5 ; et/ou
h) la substance tampon est comprise dans la composition de lyse en une concentration choisie parmi 5 mM à 100 mM, 10 mM à 80 mM et 10 mM à 50 mM.

6. Procédé selon la revendication 4 ou 5, **dans lequel** l'échantillon est mis en contact avec une composition de lyse comprenant
- au moins un tensioactif non ionique,
- au moins un polymère polyanionique hydrosoluble comprenant des monomères contenant une fonction carboxylate qui est choisi parmi le poly(acide acrylique), le poly(acide maléique) et un copolymère comprenant du poly(acide acrylique) et du poly(acide maléique), ledit polymère ayant une masse moléculaire moyenne qui se situe dans une plage de 100 000 Da à 300 000 Da, de préférence de 200 000 Da à 300 000 Da,
- au moins une substance tampon
le pH de la composition de lyse étant dans une plage choisie parmi 7,5 à 11, 7,5 à 9 et 8,0 à 8,5
pour fournir un mélange de lyse.

7. Procédé selon une ou plusieurs des revendications 1 à 6, ayant une ou plusieurs, de préférence au moins deux ou de préférence encore au moins trois des caractéristiques suivantes :
i) l'échantillon est prétraité avant la mise en contact de l'échantillon avec les additifs (a) et/ou (b) ;
ii) le mélange de lyse est incubé ;
iii) le mélange de lyse est incubé à une température choisie parmi au moins 40 °C, au moins 50 °C, au moins 90 °C et au moins 95 °C ;
iv) le mélange de lyse comprend l'échantillon lysé ;
v) le mélange de lyse est centrifugé ; et/ou
vi) le mélange de lyse est clarifié de préférence par liaison de précipités à un support solide.

8. Procédé selon une ou plusieurs des revendications 1 à 7, pour la lyse d'un échantillon fixé pour fournir un mélange de lyse qui est approprié pour être directement utilisé dans un procédé d'analyse d'acides nucléiques, dans lequel l'échantillon fixé est mis en contact avec
(a) au moins un tensioactif non ionique,
(b) au moins un polymère polyanionique hydrosoluble comprenant des monomères contenant une fonction carboxylate, ledit polymère étant
(i) un polymère constitué de monomères contenant une fonction carboxylate qui sont choisis parmi l'acide acrylique, l'acide méthacrylique et/ou l'acide maléique ou
(ii) un polymère constitué de monomères contenant une fonction carboxylate qui sont choisis parmi l'acide acrylique, l'acide méthacrylique et/ou l'acide maléique et de monomères polaires supplémentaires sans fonction carboxylate qui sont choisis parmi le lactide et/ou la vinylpyrrolidone,
de préférence ledit polymère étant le poly(acide acrylique),
et est chauffé à ≥ 85 °C pour fournir un mélange de lyse,
le procédé comprenant éventuellement
a. la mise en contact de l'échantillon biologique fixé avec
- une composition aqueuse de lyse ayant une valeur de pH qui se situe dans une plage choisie parmi 7,5 à 11, 7,5 à 9 et 8 à 8,5, comprenant
(a) au moins un tensioactif non ionique,
(b) au moins un polymère polyanionique hydrosoluble comprenant des monomères contenant une fonction carboxylate, en une concentration de 0,01 % (p/v) à 0,5 % (p/v), de préférence de 0,03 % (p/v) à 0,15 % (p/v), ledit polymère étant
(i) un polymère constitué de monomères contenant une fonction carboxylate qui sont choisis parmi l'acide acrylique, l'acide méthacrylique et/ou l'acide maléique ou
(ii) un polymère constitué de monomères contenant une fonction carboxylate qui sont choisis parmi l'acide acrylique, l'acide méthacrylique et/ou l'acide maléique et de monomères polaires supplémentaires sans fonction carboxylate qui sont choisis parmi le lactide et/ou la vinylpyrrolidone,
(c) au moins un agent tampon ; et
(d) éventuellement au moins un agent chélatant ;
et
- un support solide comprenant des groupes anioniques, de préférence des groupes carboxyle, sur la surface,
b. le chauffage à une température ≥ 90 °C, de préférence ≥ 95 °C pour fournir un mélange de lyse et éventuellement le refroidissement du mélange de lyse ;
c. la séparation du support solide du mélange de lyse restant.

9. Procédé d'amplification d'acides nucléiques, comprenant
a) la lyse d'un échantillon comprenant des acides nucléiques pour fournir un mélange de lyse qui est approprié pour être directement utilisé dans un procédé d'analyse d'acides nucléiques, l'échantillon étant mis en contact avec
(aa) au moins un tensioactif non ionique,
(ab) au moins un polymère polyanionique hydrosoluble, ledit polymère comprenant des monomères contenant une fonction carboxylate, ledit polymère étant
(i) un polymère constitué de monomères contenant une fonction carboxylate qui sont choisis parmi l'acide acrylique, l'acide méthacrylique et/ou l'acide maléique ou
(ii) un polymère constitué de monomères contenant une fonction carboxylate qui sont choisis parmi l'acide acrylique, l'acide méthacrylique et/ou l'acide maléique et de monomères polaires supplémentaires sans fonction carboxylate qui sont choisis parmi le lactide et/ou la vinylpyrrolidone,
éventuellement ledit polymère étant un polymère choisi parmi les polymères poly(acide acrylique), poly(acide maléique), poly(acide méthacrylique), poly(acide acrylique-co-acide maléique), poly(acide acrylique-co-lactide), poly(acide acrylique-co-vinylpyrrolidone), poly(acide maléique-co-lactide), poly(acide maléique-co-vinylpyrrolidone), poly(acide acrylique-co-acide maléique-co-lactide) et poly(acide acrylique-co-acide maléique-co-vinylpyrrolidone),
pour fournir un mélange de lyse, de préférence l'échantillon étant lysé selon le procédé tel que défini dans une ou plusieurs des revendications 1 à 8 ;
b) l'utilisation d'au moins une partie du mélange de lyse dans une réaction d'amplification.

10. Procédé selon la revendication 9, ayant une ou plusieurs des caractéristiques suivantes :
i) le mélange de lyse est clarifié entre les étapes a) et b) et une partie du mélange de lyse clarifié est utilisée dans une réaction d'amplification ;
ii) aucun procédé d'isolement d'acides nucléiques n'est effectué entre les étapes a) et b) ;
iii) le lysat est traité entre les étapes a) et b) par la mise en contact du lysat obtenu avec au moins une enzyme ;
iv) le lysat obtenu est utilisé pour reconstituer une composition sèche, de préférence une composition lyophilisée, qui comprend des réactifs pour la mise en oeuvre de l'amplification ; et/ou
v) la réaction d'amplification est choisie dans le groupe constitué par la réaction en chaîne par polymérase (PCR), l'amplification isotherme, la réaction en chaîne par transcription inverse et polymérase (RT-PCR), l'amplification par transcription inverse, la réaction en chaîne par polymérase quantitative en temps réel (qPCR), le séquençage d'ADN ou d'ARN, la transcription inverse, la LAMP (amplification isotherme induite par boucle), la RPA (amplification par recombinase et polymérase), la tHDA (amplification hélice-dépendante), la NEAR (réaction d'amplification par enzyme de coupure), la TMA (amplification induite par transcription) et la NASBA (amplification basée sur les séquences d'acides nucléiques), la réaction en chaîne par polymérase allèle-spécifique, l'assemblage par cycles par polymérase (PCA), la réaction en chaîne par polymérase asymétrique, la réaction en chaîne par polymérase linéaire après exponentielle (LATE-PCR), l'amplification hélicase-dépendante (HDA), la réaction en chaîne par polymérase à démarrage à chaud, la réaction en chaîne par polymérase inter-séquence-spécifique (ISSR), la réaction en chaîne par polymérase inverse, la réaction en chaîne par polymérase induite par ligature, la réaction en chaîne par polymérase méthylation-spécifique (MSP), la réaction en chaîne par polymérase multiplexe, la réaction en chaîne par polymérase nichée et la réaction en chaîne par polymérase en phase solide.

11. Solution de lyse appropriée pour fournir un mélange de lyse qui peut être directement utilisé dans une amplification d'acides nucléiques, comprenant
(a) au moins un tensioactif non ionique,
(b) au moins un polymère polyanionique hydrosoluble comprenant des monomères contenant une fonction carboxylate, l'au moins un polymère polyanionique hydrosoluble ou le mélange de polymères polyanioniques hydrosolubles étant compris dans la solution de lyse en une concentration de 0,01 % (p/v) à 1 % (p/v) et ledit polymère étant
(i) un polymère constitué de monomères contenant une fonction carboxylate qui sont choisis parmi l'acide acrylique, l'acide méthacrylique et/ou l'acide maléique ou
(ii) un polymère constitué de monomères contenant une fonction carboxylate qui sont choisis parmi l'acide acrylique, l'acide méthacrylique et/ou l'acide maléique et de monomères polaires supplémentaires sans fonction carboxylate qui sont choisis parmi le lactide et/ou la vinylpyrrolidone,
éventuellement ledit polymère étant un polymère choisi parmi les polymères poly(acide acrylique), poly(acide maléique), poly(acide méthacrylique), poly(acide acrylique-co-acide maléique), poly(acide acrylique-co-lactide), poly(acide acrylique-co-vinylpyrrolidone), poly(acide maléique-co-lactide), poly(acide maléique-co-vinylpyrrolidone), poly(acide acrylique-co-acide maléique-co-lactide) et poly(acide acrylique-co-acide maléique-co-vinylpyrrolidone).

12. Solution de lyse selon la revendication 11, comprenant de plus
(c) au moins une enzyme protéolytique ;
(d) au moins un agent chélatant pour des cations divalents ; et/ou
(e) au moins une substance tampon.

13. Solution de lyse selon la revendication 11 ou 12, dans laquelle l'au moins un polymère polyanionique hydrosoluble comprenant des monomères contenant une fonction carboxylate est choisi parmi le poly(acide acrylique), le poly(acide maléique) et un copolymère comprenant du poly(acide acrylique) et du poly(acide maléique) et dans laquelle ledit polymère a une masse moléculaire moyenne qui se situe dans une plage de 100 000 Da à 300 000 Da, de préférence de 200 000 Da à 300 000 Da, et le pH de la solution de lyse étant dans une plage choisie parmi 7,5 à 11, 7,5 à 9 et 8,0 à 8,5.

14. Solution de lyse selon une ou plusieurs des revendications 11 à 13, ayant une ou plusieurs, de préférence au moins deux, au moins trois, au moins quatre et de préférence encore au moins cinq des caractéristiques suivantes :
a) l'au moins un polymère polyanionique hydrosoluble a une ou plusieurs des caractéristiques telles que définies dans la revendication 3 ;
b) l'au moins tensioactif non ionique ou le mélange de tensioactifs non ioniques est compris dans la solution de lyse en une concentration choisie dans une plage de 0,05 % (v/v) à 5 % (v/v), de 0,1 % (v/v) à 2 % (v/v), de 0,2 % (v/v) à 1,5 % (v/v) et de 0,4 % (v/v) à 1 % (v/v) ;
c) le tensioactif non ionique ou le mélange de tensioactifs non ioniques est choisi dans le groupe constitué par les éthers d'alcools gras et de polyoxyéthylène, les éthers d'alkylphényle et de polyoxyéthylène, les copolymères séquencés de polyoxyéthylène-polyoxypropylène, les alkylglucosides et les produits d'éthoxylation d'alkylphénols, de préférence les éthers d'alcools gras et de polyoxyéthylène, les polysorbates et/ou les éthers d'alkylphényle et de polyoxyéthylène ;
d) l'au moins un polymère polyanionique hydrosoluble ou le mélange de polymères polyanioniques hydrosolubles est compris dans la solution de lyse en une concentration qui est choisie dans une plage de 0,02 % (p/v) à 0,5 % (p/v), de 0,03 % (p/v) à 0,3 % (p/v), de 0,04 % (p/v) à 0,2 % (p/v), de 0,05 % (p/v) à 0,15 % (p/v) et de 0,05 % (p/v) à 0,1 % (p/v) et, de préférence, l'au moins un polymère polyanionique hydrosoluble étant choisi parmi le poly(acide acrylique), le poly(acide maléique) et un copolymère comprenant du poly(acide acrylique) et du poly(acide maléique) et de préférence encore étant ou comprenant du poly(acide acrylique) ;
e) l'au moins un polymère polyanionique hydrosoluble qui est ou comprend du poly(acide acrylique) a une masse moléculaire moyenne qui se situe dans une plage de 100 000 Da à 300 000 Da, de préférence de 200 000 Da à 300 000 Da ;
f) l'enzyme protéolytique est une protéinase thermophile ;
g) l'au moins un agent chélatant est choisi dans le groupe constitué par l'acide diéthylènetriaminepentaacétique (DTPA), l'acide éthylènedinitrilotétraacétique (EDTA), l'acide éthylèneglycoltétraacétique (EGTA) et la *N,N*-bis(carboxyméthyl)glycine (NTA) ;
h) l'au moins un agent chélatant est compris dans la solution de lyse en une concentration choisie dans une plage de 0,5 mM à 5 mM, de 0,75 mM à 2 mM et de 1 mM à 1,5 mM;
i) la substance tampon est utilisée dans la solution de lyse de façon à ce que le pH de la solution soit choisi dans une plage de 7,5 à 11, de 7,5 à 9 et de 8,0 à 8,5 ;
j) la substance tampon est comprise dans la solution de lyse en une concentration choisie dans une plage de 5 mM à 100 mM, de 10 mM à 80 mM et de 10 mM à 50 mM ; et/ou
k) la solution de lyse est une solution de lyse comprenant
- en tant qu'additif (a) un mélange de deux détergents non ioniques, chaque détergent non ionique étant compris en une concentration de 0,2 % (v/v) à 0,6 % (v/v), de préférence de 0,4 % (v/v) à 0,5 % (v/v) ;
- en tant qu'additif (b), un polymère polyanionique hydrosoluble, qui de préférence est ou comprend du poly(acide acrylique), en une concentration de 0,01 % (p/v) à 0,5 % (p/v), de préférence de 0,03 % (p/v) à 0,15 % (p/v) ;
- éventuellement une enzyme protéolytique telle que la protéinase K en tant qu'additif (c) ;
- en tant qu'additif (d) de l'EDTA en une concentration au-dessous de 1,5 mM ; et
- en tant qu'additif (e) une substance tampon, de préférence du TRIS, en une concentration choisie parmi 7,5 mM à 50 mM.

15. Solution de lyse telle que définie dans l'une quelconque des revendications 11 à 14, qui est mélangée avec un échantillon contenant des acides nucléiques.
